# EUROPEAN PATENT APPLICATION

(11) **EP 4 759 356 A1**
(43) Date of publication of application: **17.06.2026**
(21) Application number: 24220274.5
(22) Date of filing: 16.12.2024
(51) Int. Cl.: A61N 5/10, A61D 99/00, G21K 5/04

(54) **TRANSPORTABLE BEAMLINE FOR ION BEAM IRRADIATION**

(71) Applicant: Ludwig-Maximilians-Universität München, 80539 München (DE)
(72) Inventor: Parodi, Katia, 81375 Munich (DE); Kurichiyanil, Neeraj, 64285 Darmstadt (DE); Pinto, Marco, 70565 Stuttgart (DE); Bortfeldt, Jonathan, 93055 Regensburg (DE); Huang, Ze, 80939 Munich (DE)
(74) Representative: Lucke, Andreas

(57) **Abstract**

Disclosed herein is a transportable beamline (100, 200) for ion beam irradiation, in particular small animal ion beam irradiation, and a method (700) of aligning a transportable beamline. The beamline (100, 200) is configured to receive an ion beam (102), in particular a proton beam, from an ion beam source and to guide the ion beam (102) along a beam path (104) to a target point (106) that is to be irradiated. The beamline (100, 200) comprises: an energy degrader (108, 500) for decelerating ions in the ion beam (102); a collimator (110, 400) for collimating the ion beam (102); a focusing assembly (112) for focusing the ion beam (102), the focusing assembly (112) being arranged downstream of the energy degrader (108, 500) and the collimator (110, 400) along the beam path (104) for the ion beam (102) and comprising two or more quadrupole magnets (112A-C) arranged along the beam path (104); and a common base structure (114) to which at least the collimator (110, 400) and the focusing assembly (112) are mounted, the common base structure (114) being configured to preserve relative alignment between the collimator (110, 400) and the two or more quadrupole magnets (112A-C) during transport of the beamline (100, 200), wherein some or all of said two or more quadrupole magnets (112A-C) are removably mounted to the common base structure (114) via a respective kinematic mount (116, 300).

## Description

### FIELD OF THE INVENTION

The present invention is in the field of medical physics and relates to a transportable beamline for ion beam irradiation, in particular small animal ion beam irradiation, as well as to a method of aligning such a transportable beamline.

### BACKGROUND

Particle therapy employs accelerated particles such as protons or other ions to irradiate tissue for treatment and is for example used for treating cancer. As compared to other forms of radiotherapy such as for example photon radiotherapy, particle therapy and in particular proton therapy may allow for a highly selective treatment of a localized target region with minimal entrance and distal doses outside the target region, thereby reducing damage to healthy tissue. This is due to the nature of the energy-loss mechanism of ions in matter, which results in the highest dose being deposited in a highly localized region, the so-called Bragg peak.

Clinical facilities for the treatment of patients, which typically provide proton beams with a particle energy between 70 MeV and 250 MeV at spot sizes (e.g., σ) of around 3 mm to 5 mm, are often unsuitable for preclinical studies on small animals such as mice as the latter may require lower particle energies and smaller spot sizes. While small animal irradiation platforms are readily available for photon radiotherapy, similar devices for proton therapy remain an emerging as well as challenging field of research. Current practices in small animal proton therapy are mostly limited to the usage of energy degraders and beam collimators for adapting clinical beams to small animal proton therapy platforms, see, e.g., E. Ford et al., Physics in Medicine and Biology, 62(1):43-58, December 2016 and M. M. Kim et al., Physics in Medicine & Biology, 64(13):135013, July 2019*.* Such "passive" approaches, however, while being straight forward to implement and facility independent, may produce significant amounts of secondary radiation and offer limited flexibility in terms of particle fluence and spot size. Nuclear medicine research facilities or other radiation facilities may also be considered for preclinical studies but also typically have unsuitable beams (e.g., due to too high energy and/or size) for such applications.

To address these issues, the present inventors and others have proposed a novel "active" system for small animal proton irradiation that additionally employs quadrupole magnets for active magnetic focusing of the beam, see for example K. Parodi et al., Acta Oncologica, 58(10):1470-1475, October 2019*.* This system is intended as a mobile platform for use at existing clinical facilities or other facilities providing higher-energy proton beams. As a result of its increased complexity, however, making such a system suitable for installation at a clinical or research facility poses considerable design challenges. For example, beam time may be limited and often it may only be possible to have the system installed temporarily for a limited amount of time due to competing intended use of the expensive beam, thus requiring frequent re-installation of the system and alignment to the beam.

### SUMMARY OF THE INVENTION

The object of the present invention is therefore to provide a transportable beamline for small animal proton beam irradiation that allows for rapid installation at a higher-energy proton source such as a clinical proton source.

This object is met by a transportable beamline for ion beam irradiation and a method of aligning a transportable beamline as set out in the independent claims. Examples thereof are detailed in the dependent claims.

Any feature or combination of features described herein with regard to the beamline according to the present invention may also be present for the method according to the present invention. Additionally or alternatively, any feature or combination of features described herein with regard to the method according to the present invention may also be present for the beamline according to the present invention. For the sake of brevity and to avoid unnecessary repetition, certain features or combination of feature may therefore only be described once with regard to either the beamline or the method, but may likewise apply to the other one of the beamline and the method.

The present invention provides a transportable beamline for ion beam irradiation, in particular small animal ion beam irradiation, that is configured to receive an ion beam, in particular a proton beam, from an ion beam source and to guide the ion beam along a beam path to a target point that is to be irradiated. The beamline comprises an energy degrader for decelerating ions in the ion beam, a collimator for collimating the ion beam and a focusing assembly for focusing the ion beam. The focusing assembly is arranged downstream of the energy degrader and the collimator along the beam path for the ion beam and comprises two or more quadrupole magnets arranged along the beam path. The beamline further comprises a common base structure to which at least the collimator and the focusing assembly are mounted. The common base structure is configured to preserve relative alignment between the collimator and the two or more quadrupole magnets during transport of the beamline. Some or all of said two or more quadrupole magnets are removably mounted to the common base structure via a respective kinematic mount.

The beamline is for use with an external ion beam source, which generates the ion beam and provides the ion beam to the beamline (e.g., an entrance aperture such as an entrance window thereof). The types of ions as well as the ion beam source are not particularly limited and the beamline according to the present invention may be adapted for use with various types of ions and/or ion beam sources. The ions may for example be any type of ion used for medical therapy and/or biomedical research, in particular, but not limited to, protons, helium ions, carbon ions, oxygen ions and/or iron ions. The ion beam source may in particular be a clinical ion beam source (e.g., a clinical proton source), i.e., an ion beam source as commonly used in medical particle therapy, e.g., for cancer treatment. The beamline may for example be configured to receive an ion beam of ions having an initial energy (e.g., as provided at an outlet of the ion beam source) of between 10 MeV/mᵤ and 500 MeV/mᵤ, in some examples between 15 MeV/mᵤ and 250 MeV/mᵤ and in some examples between 20 MeV/mᵤ and 100 MeV/mᵤ with mᵤ being the mass of the ions in atomic mass units (u) (i.e., for protons (mᵤ = 1) between 10 MeV and 500 MeV, in some examples between 15 MeV and 250 MeV and in some examples between 20 MeV and 100 MeV). The beamline may for example be configured to receive an ion beam of ions having an initial energy of between 50 MeV/mᵤ and 100 MeV/mᵤ (which may, e.g., be a range of typical lowest energies that can be provided by ion beam sources at clinical facilities) and in one example between 70 MeV/mᵤ and 100 MeV/mᵤ. Additionally or alternatively, the beamline may for example be configured to receive an ion beam of ions having an initial energy of between 30 MeV/mᵤ and 50 MeV/mᵤ (which may, e.g., be a range of typical lowest energies that can be provided by ion beam sources at preclinical facilities such as radioisotope cyclotrons) and in one example between 35 MeV/mᵤ and 40 MeV/mᵤ The beamline may be configured to be installed at the ion beam source. For this, the beamline may for example be mounted or configured to be mounted to a supporting platform such as an optical table, e.g. as detailed below.

The beamline may be used to irradiate a target with the ion beam, e.g., by placing the target at or in the vicinity of the target point. The types of targets that may be irradiated by the beamline are not particularly limited and may comprise both biological targets (e.g., humans, animals or parts thereof such as tissue samples) as well as non-biological targets. The beamline may in particular be configured for the irradiation of small animals as commonly used in biomedical research, for example mice.

The beamline is configured to guide the ion beam along the beam path to the target point that is to be irradiated. The beam path may be defined by the beamline and may, e.g., correspond to the path along which the ion beam is supposed to travel during use of the beamline (e.g., the central axis of the beamline and/or of components thereof such as an entrance aperture, the degrader, the collimator, the focusing assembly and/or an exit aperture). The beam path may for example extend from an entrance aperture (e.g., an entrance window) of the beamline to an exit aperture (e.g., an exit window) of the beamline.

As used herein, a direction along the beam path may be referred to as the longitudinal direction, length direction or Z direction. The terms "upstream" and "downstream" (or similar relative terms with respect to the beam path) may refer to an element or component that is closer to the ion beam source (farther away from the target point) and farther away from the ion beam source (closer to the target point), respectively, than another element or component. A direction perpendicular to the beam path may be referred to as a lateral or transverse direction, wherein a direction perpendicular to the beam path and to the common base structure or supporting platform on which the beamline is (to be) mounted may be referred to as the vertical direction, height direction or Y direction and a direction perpendicular to the beam path and the Y direction may be referred to as the horizontal direction, width direction or X direction. It is, however, to be noted that this is not intended to imply a particular orientation of the beamline with respect to the direction of gravity (i.e., the Y/vertical direction may, but does not necessarily have to coincide with the direction of gravity). As far as technically feasible, the beamline may have an arbitrary orientation with respect to the direction of gravity. Relative terms such as "bottom" or "lower" may refer to an element or component that is further in the direction towards (e.g., closer to) the common base structure or the supporting platform (as, e.g., seen from the beam path) than another element or component, whereas relative terms such as "top" or "upper" may refer to an element or component that is further in the direction towards (e.g., closer to) the beam path (as, e.g., seen from the common base structure or the supporting platform) than another element or component (again without intending to imply any particular orientation with respect to the direction of gravity by the use of such terms).

The beamline is transportable, for example such that the beamline can be transported within an ion beam source facility (e.g., from a storage room to the ion beam source or vice-versa) or between two ion beam source facilities. As used herein, the term "transportable beamline" may refer to a beamline having overall physical dimensions and/or an overall weight so as to be compatible with standard road transport and/or typical door sizes and/or floor load ratings (e.g., of a clinical environment such as a clinical ion beam source facility). In some examples, the beamline may be suitable for transport by hand (i.e., without requiring lifting or transport aids such as a fork lift). A total length of the beamline (e.g., without the supporting platform, for example from the entrance aperture to the exit aperture of the beamline) along the beam path may for example be less than 2 m, preferably less than 1.5 m, most preferably less than 1.2 m and in some examples less than 1.0 m. A total extent of the beamline (e.g., without the supporting platform) perpendicular to the beam path (e.g., a height in the Y direction and/or a width in the X direction) may for example be less than 1.5 m, preferably less than 1.0 m, most preferably less than 0.8 m and in some examples less than 0.5 m. A total weight of the beamline (e.g., without the supporting platform) may for example be less than 250 kg, preferably less than 200 kg, most preferably less than 150 kg and in some examples less than 100 kg. The physical dimensions and/or weight of the beamline may for example be reduced by avoiding or minimizing the use of heavy radiation shielding (e.g., employing low- and/or short-lived-activation materials such as graphite instead) and/or of electromagnets (e.g., using permanent magnets instead).

The energy degrader may be configured to decelerate the ions in the ion beam from an initial energy to a target energy, e.g., as detailed below. The present invention is not limited to any particular means for decelerating the ions and, as far as technically feasible, may be implemented with any type of energy degrader known in the art, for example a passive degrader comprising one or more degrading members (e.g., as detailed below), and/or an active degrader, which may, e.g., employ electromagnetic fields for decelerating the ions. The energy degrader may be arranged upstream of the collimator and the focusing assembly.

In some examples, the energy degrader may comprise one or more degrading members arranged in the beam path or arrangeable in the beam path (e.g., configured to be inserted into and removed from the beam path) for the ion beam to pass therethrough. Each degrading member may be configured to decelerate the ions passing therethrough, wherein the energy loss of the ions may for example depend on a thickness of the degrading member (along the beam path). The one or more degrading members may for example be made of (i.e., comprise or consist of) graphite, beryllium, lithium, a polycarbonate, water and/or aluminum. Preferably, the one or more degrading members comprise or consist of boron carbide (B₄C). For example, the energy degrader may comprise one or more disk-shaped degrading members (e.g., counter rotating disks of varying thickness) and/or one or more wedge-shaped degrading members (e.g., wedges of varying thickness). In one example, the energy degrader comprises a pair of wedge-shaped degrading members facing each other (e.g., such that a length of the beam path through the wedge-shaped degrading members is the same or substantially the same irrespective of the lateral positioning of the pair of degrading members relative to the beam path).

The energy degrader may be configured to decelerate the ions having an initial energy as commonly provided by higher-energy ion beam sources such as clinical ion beam source facilities (e.g., an initial energy as listed above, for example between 15 MeV/mᵤ and 250 MeV/mᵤ , in some examples between 20 MeV/mᵤ and 100 MeV/mᵤ with mᵤ denoting the mass of the ions in atomic mass units u) to a target energy suitable for small animal irradiation (e.g., a target energy between 10 MeV/mᵤ and 60 MeV/mᵤ). For example, the energy degrader may be configured to decelerate ions in the ion beam having an initial energy between 50 MeV/mᵤ and 100 MeV/mᵤ (range of typical lowest energies that can be provided by ion beam sources at clinical facilities with the lowest possible energy preferably being used for operating the beamline/deriving the degraded beam), in some examples between 70 MeV/mᵤ and 100 MeV/mᵤ, to a target energy between 10 MeV/mᵤ and 60 MeV/mᵤ, preferably between 20 MeV/mᵤ and 50 MeV/mᵤ (i.e., for at least one initial energy in the range between 50 MeV/mᵤ and 100 MeV/mᵤ (e.g., 70 MeV/mᵤ), the ions in the ion beam may be decelerated to a (lower) target energy within the range between 10 MeV/mᵤ and 60 MeV/mᵤ or preferably 20 MeV/mᵤ and 50 MeV/mᵤ). Additionally or alternatively, the energy degrader may be configured to decelerate ions in the ion beam having an initial energy between 30 MeV/mᵤ and 50 MeV/mᵤ (range of typical lowest energies that can be provided by ion beam sources at preclinical facilities with the lowest possible energy preferably being used for operating the beamline/deriving the degraded beam), in some examples between 35 MeV/mᵤ and 40 MeV/mᵤ, to a target energy between 10 MeV/mᵤ and 40 MeV/mᵤ, preferably between 10 MeV/mᵤ and 30 MeV/mᵤ.

In a preferred embodiment, the energy degrader is an adjustable energy degrader that is configured to decelerate ions in the ion beam from a fixed initial energy to an adjustable target energy. The adjustable degrader may be configured to decelerate ions having an initial energy within the ranges as listed above to a (lower) adjustable target energy, e.g., within the ranges as listed above. The adjustable degrader may for example be configured to decelerate ions having an initial energy between 50 MeV/mᵤ and 100 MeV/mᵤ, in some examples between 70 MeV/mᵤ and 100 MeV/mᵤ (i.e., for at least one given value within this range, e.g., 70 MeV/mᵤ) to a (lower) target energy that is adjustable from 30 MeV/mᵤ to 40 MeV/mᵤ, preferably from 20 MeV/mᵤ to 50 MeV/mᵤ, in one example from 10 MeV/mᵤ to 60 MeV/mᵤ. The energy degrader may for example be configured to adjust an amount of material that the ions pass through (e.g., a length of the beam path through the one or more degrading members), for example by moving the one or more degrading members relative to the beam path. For example, the energy degrader may comprise a pair of wedge-shaped degrading members as described above and may be configured to adjust a lateral spacing between the wedge-shaped degrading members to adjust the target energy.

The collimator may be configured to collimate the ion beam, for example such that the ion beam has a particular lateral extent (e.g., width or diameter) and/or a particular divergence (e.g., opening angle). For this, the collimator may for example comprise one or more apertures, e.g., as detailed below. The collimator may be configured to collimate the ion beam in the same way (e.g., to the same lateral extent and/or the same divergence) irrespective of the ion beam as received by the beamline and/or of the ion beam source. The collimator may thus serve as an internal reference (e.g., an internal "beam source") so to provide an ion beam having the same well-defined beam parameters independent of the ion beam source facility at which the beamline is used and independent of the alignment of the beamline to the ion beam source. The collimator (or at least a part thereof) may be arranged between the energy degrader and the focusing assembly.

The focusing assembly may be configured to focus the ion beam, for example to a focus point (e.g., a point of smallest lateral extent), which may or may not coincide with the target point (for example, the target point may be chosen to not lie at the focus point but rather at a point where the beam has a more uniform aspect ratio, e.g., an aspect ratio closest to one). The focusing assembly comprises at least two quadrupole magnets, preferably at least three quadrupole magnets (e.g., an upstream quadrupole magnet, a central quadrupole magnet and a downstream quadrupole magnet arranged along the beam path). The magnetic axes of the quadrupole magnets (e.g., the axes where the magnetic field vanishes) may be aligned with the beam path (i.e., coincide with a portion of the beam path), e.g., using the method according to the present invention. A quadrupole magnet may act as a magnetic lens for the ion beam, wherein the magnetic field of the quadrupole magnet may focus the ion beam in one transverse plane (e.g., the X-Z-plane) while defocusing the ion beam in the orthogonal transverse plane (e.g., the Y-Z-plane). The quadrupole magnets of the focusing assembly may be arranged so as to collectively focus the ion beam in both transverse planes. For example, for each of the two transverse planes, the focusing assembly may comprise at least one quadrupole magnet that is configured to focus the ion beam in the respective transverse plane. In one example, the quadrupole magnets are arranged in alternating focusing order (e.g., focusing-defocusing-focusing-...). In one example, the focusing assembly may be configured to manipulate the ion beam so as to generate a fan-like beam and/or a parallel beam.

At least the collimator and the focusing assembly (i.e., the quadrupole magnets) and, optionally, the energy degrader are mounted to a common base structure. The common base structure may serve as an internal reference frame to preserve the relative alignment between the collimator and the two or more quadrupole magnets, in particular during transport of the beamline. The collimator and the focusing assembly may be the most critical components of the beamline in terms of alignment, whereas other components such as the energy degrader, in particular those arranged upstream of the collimator or downstream of the focusing assembly, may be less critical and thus need not (but optionally may) be mounted to the common base structure. The common base structure may be configured for mounting to a supporting platform such as an optical table, e.g., as detailed below.

The common base structure may be configured to preserve the relative alignment between the collimator and the two or more quadrupole magnets with an accuracy of (i.e., limit relative displacement or drift between the respective components to) no more than 100 µm, preferably no more than 50 µm, most preferably no more than 30 µm, in one example no more than 20 µm and in one example no more than 15 µm. For this, the common base structure may comprise or consist of materials with high mechanical stability and/or low thermal expansion. A coefficient of linear thermal expansion (e.g., in the X-, Y-, and/or Z-direction) of the common base structure may for example be no more than 15 · 10⁻⁶/K, in some examples no more than 12.5 · 10⁻⁶/K, preferably no more than 10 · 10⁻⁶/K, in one example no more than 5 · 10⁻⁶/K and in one example no more than 3 · 10⁻⁶/K. The common base structure may for example comprise or consist of one or more of granite, a carbon fiber compound, an iron-nickel alloy (e.g., invar), tantalum, tungsten, titanium, ferritic stainless steel, iron (e.g., cast or forged iron), marble, glass and porcelain.

In a preferred embodiment, the common base structure is formed of (e.g., is or consists of) a single piece of material (i.e., may be integrally formed of or from a single material). The common base structure may in particular be formed of a single piece of material having a coefficient of linear thermal expansion of no more than 15 · 10⁻⁶/K, in some examples no more than 12.5 · 10⁻⁶/K, preferably no more than 10 · 10⁻⁶/K, in one example no more than 5 · 10⁻⁶/K and in one example no more than 3 · 10⁻⁶/K. The common base structure may for example be a single piece of granite, a carbon fiber compound, an iron-nickel alloy and/or one of the other materials listed above.

Some or preferably all of the two or more quadrupole magnets in the focusing assembly are mounted to the common base structure by means of a respective kinematic mount. The respective quadrupole magnets may be mounted via the respective kinematic mount to the common base structure directly (e.g., with no additional elements or components arranged therebetween) or indirectly (e.g., with one or more additional elements or components such as, e.g., a translation stage or another kinematic mount arranged therebetween). The mounting may be removable, i.e., such that the respective quadrupole magnet can be removed from the common base structure (e.g., by removing a holder portion of the kinematic mount holding the magnet from a mount portion of the kinematic mount coupled to the common base structure).

As used herein, a kinematic mount may refer to a mount that is configured to hold a first element or component (e.g., a quadrupole magnet or a housing or holder for the quadrupole magnet) in place relative to a second element or component (e.g., the common base structure) by kinematic coupling (also referred to as kinematic constraint), i.e., fixing the first element without over-constraining the first element. Put differently, a kinematic mount may have as many points of contact (e.g., between a first portion of the kinematic mount coupled to the first element and a second portion of the kinematic mount coupled to the second element) as the number of motional degrees of freedom that are to be constrained. This may allow for precise positioning of the two elements with respect to each other without introducing strain and/or instabilities and thus with high repeatability. Thereby, the respective quadrupole magnets can easily be removed from the beamline (e.g., for transport) and later re-positioned on the common base structure with high accuracy so as to maintain alignment relative to the collimator and the other quadrupole magnets. This may greatly facilitate installation and alignment of the beamline to an external ion source.

Some or all of the kinematic mounts may be configured to constrain all six motional degrees of freedom of the respective quadrupole magnet (i.e., its three translational degrees of freedom as well as its three rotational degrees of freedom). Accordingly, the respective kinematic mount may have six points of contact. Some or all of the kinematic mounts may for example be Kelvin-type kinematic mounts (e.g., having three mount points at which spherical contact surfaces rest on a concave tetrahedron, a V-shaped groove and a flat surface, respectively, or vice-versa) or Maxwell-type kinematic mounts (e.g., having three mount points at which curved contact surfaces rest on V-shaped grooves or vice-versa).

Preferably, some or all of the kinematic mounts are adjustable kinematic mounts that are configured to adjust a position and/or orientation of the respective quadrupole magnet relative to the common base structure, e.g., by translating and/or rotating the respective quadrupole magnet along/about one or more axes, preferably three axes (i.e., in all directions/all six motional degrees of freedom).

Some or all of the adjustable kinematic mounts may for example comprise a mount portion coupled to (e.g., attached to or mounted to) the common base structure (e.g., directly without any intermediate elements or components therebetween or indirectly via one or more intermediate elements or components such as a translation stage) and a holder portion holding the respective quadrupole magnet (e.g., forming a holder, housing or case for the magnet). The mount portion and the holder portion may be kinematically (and, optionally, removably) coupled via a set of kinematic mount points (each of which may comprise or form one or more points of contact between the mount and holder portions, e.g., as described above). Preferably, the set of kinematic mount points comprises three mount points. The set of kinematic mount points may be or comprise one or both of the first set of kinematic mount points on the mount portion and the second set of kinematic mount points on the intermediate portion described below. The kinematic mount points may be moveable (e.g., in one or more directions, preferably in all three directions) so as to allow for adjusting the position and the orientation of the respective quadrupole magnet with respect to the mount portion (and thereby the common base structure), preferably in all six motional degrees of freedom.

The mount portion may comprise a set of height-adjustable pins, in particular height-adjustable threaded pins. Preferably, the mount portion comprises three height-adjustable pins. The height-adjustable pins (e.g., a top surface thereof) may form a first set of kinematic mount points (e.g., three mount points) of the adjustable kinematic mount (e.g., each pin may form a respective mount point). The height-adjustable pins may be configured to be extended from and/or retracted towards the mount portion along a first direction for moving the respective mount point in the first direction (e.g., by rotation of the threaded pins). This may allow for adjusting the position of the respective quadrupole magnet in the first direction (e.g., away from or towards the mount portion) and the orientation of the respective quadrupole magnet about a second direction and a third direction perpendicular to the first direction (i.e., one translational degree of freedom and two rotational degrees of freedom). The first and second directions may for example be lateral directions (i.e., perpendicular to the beam path), in particular the vertical/Y direction and the horizontal/X direction, respectively, and the third direction may for example be the longitudinal direction (e.g., along the beam path).

Additionally or alternatively, the mount portion may comprise a bottom part that is coupled to the common base structure (e.g., directly or indirectly) and an upper part that forms and/or supports the first set of mount points. The upper part may be slidably arranged on the bottom part, for example such that the upper part may be moved relative to the bottom part in one or both of the second and third directions (e.g., in the horizontal/X direction and/or the longitudinal direction). This may allow for adjusting the position of the respective quadrupole magnet in the second and third directions as well as the orientation of the respective quadrupole magnet about the first direction (e.g., the vertical/Y direction), i.e., the remaining two translational degrees of freedom and the remaining rotational degree of freedom. The bottom and upper parts may further be configured to be locked to each other to prevent sliding of the upper part relative to the bottom part. For this, the mount portion may comprise one or more locking means such as screws and/or clamps.

The beamline may further comprise a mechanical alignment template for aligning the first set of mount points. The alignment template may have a plurality of precision holes and/or recesses (e.g., on its bottom surface) that are arranged in a pattern corresponding to nominal positions of at least a subset (e.g., at least one, preferably at least two, in some examples of all) of the first set of mount points for each of the adjustable kinematic mounts that comprise a mount portion with height-adjustable pins. Each of the precision holes and/or recesses may be configured to receive a respective one of the height-adjustable pins. Thereby, the first set of mount points of the respective adjustable kinematic mounts may be aligned mechanically with respect to each other (e.g., by placing the height-adjustable pins in the corresponding holes/recesses). The precision holes and/or recesses may be precision-machined so as to match a shape (e.g., a diameter) of the respective height-adjustable pins, for example with a tolerance of at most 100 µm, preferably at most 50 µm, most preferably at most 30 µm, in one example at most 20 µm and in one example at most 15 µm. In some examples, the alignment template may comprise one or more reference markers for aligning the alignment template within the beamline (e.g., for absolute alignment of the first set of mount points with respect to the beam path in addition to the relative alignment of the first set of mount points with respect to each other). The reference markers may for example be or comprise optical reference markers for alignment to an alignment laser beam, e.g., one or more holes for the alignment laser beam to pass through. Additionally or alternatively, the reference markers may also be or comprise mechanical reference markers, e.g., precision holes/recesses and/or pins for mechanical alignment to a corresponding counterpart.

In some examples, some or all of the adjustable kinematic mounts may comprise an intermediate portion that is arranged between the mount and holder portions. In such examples, the mount portion and the intermediate portion may be kinematically (and, optionally, removably) coupled at the first set of moveable kinematic mount points (on the mount portion, e.g., formed by the height-adjustable pins). The intermediate portion and the holder portion may in turn be kinematically (and, optionally, removably) coupled at a second set of moveable kinematic mount points (preferably three mount points) on the intermediate portion. Put differently, the respective kinematic mounts may comprise a pair of kinematic mountings/couplings stacked in two layers or stages (between the mount and intermediate portions and between the intermediate and holder portions), thus each forming a two-stage kinematic mount. The second set of moveable kinematic mount points may be used for adjusting the position and the orientation of the respective quadrupole magnet with respect to the intermediate portion (preferably in all six motional degrees of freedom). This may for example allow for aligning a magnetic axis of the respective quadruple magnet relative to the intermediate portion independently (e.g., prior to) aligning the quadrupole magnets (e.g., their magnetic axes) relative to each other and/or to the beam path using the first set of mount points.

The intermediate portion may comprise a base (bottom part), which may be arranged on the first set of mount points. The intermediate portion may further comprise a tip/tilt plate (upper part) that forms and/or supports the second set of mount points. The tip/tilt plate may be moveably supported by the base so as to be tiltable and translatable with respect to the base in three directions (e.g., about/along the first, second and third directions when placed on the mount portion), i.e., adjustable in all six motional degrees of freedom. The tip/tilt plate may for example be supported by the base via a plurality of screws, in particular micrometer screws, configured to be rotated for tilting and/or translating the tip/tilt plate with respect to the base. Similar to the mount portion, the base and the tip/tilt plate of the intermediate portion may be configured to be locked to each other to prevent the tip/tilt plate for tilting and translating with respect to the base (e.g., to fix the magnetic axis of the respective quadrupole magnet relative to the intermediate portion).

The focusing assembly may be configured to adjust the focus point of the ion beam (i.e., focus the ion beam to an adjustable focus point), e.g., move the focus point longitudinally along the beam path. For this, the focusing assembly may have a variable focal length (e.g., in two transverse planes). The focal length of the focusing assembly may depend on the energy of the ions in the ion beam and varying the focal length may thus for example allow for focusing ion beams of different energy onto the same focus point. In one example, one or more of the quadrupole magnets may be electromagnets (e.g., superconducting and/or normal-conducting electromagnets) having a variable magnetic field strength for varying the focal length of the focusing assembly.

Preferably, some or all of the quadrupole magnets are mounted to a translation stage, in particular an automated computer-controller translation stage, on the common base structure. The translation stage may be configured to move the respective magnet longitudinally along the beam path relative to the common base structure. This may allow for varying the focal length of the focusing assembly also for magnets with a fixed magnetic field strength, in particular permanent magnets, e.g., by varying the drift lengths between the quadrupole magnets in the focusing assembly. For example, at least two of the quadrupole magnets (e.g., the two upstream most quadrupole magnets) may be mounted to a respective translation stage to allow for individually moving the respective quadrupole magnet relative to the other quadrupole magnets along the beam path.

In a preferred embodiment, the focusing assembly comprises an upstream quadrupole magnet, a central quadrupole magnet and a downstream quadrupole magnet arranged along the beam path and a first translation stage to which both the upstream quadrupole magnet and the central quadrupole magnet are mounted. The first translation stage may be configured to move the upstream and central quadrupole magnets longitudinally along the beam path relative to the collimator and the downstream quadrupole magnet. This may allow for adjusting an upstream drift length between the collimator and the upstream quadrupole magnet and a downstream drift length between the central quadruple magnet and the downstream quadrupole magnet while maintaining a central drift length between the upstream and central quadrupole magnets (as well as a total length of the beamline). Adjusting the upstream and downstream drift lengths of a quadrupole triplet may be sufficient for moving the focus point longitudinally along the beam path and thereby adjusting the focus point to different energies of the ions.

In some examples, the focusing assembly may additionally or alternatively be configured to move the target point perpendicular to the beam path, e.g., by deflecting the ion beam laterally. This may for example be achieved by moving one or more quadrupole magnets laterally (e.g., in the X- and/or Y-directions), e.g., by mounting one or more quadrupole magnets to a translation stage, in particular an automated computer-controller translation stage, on the common base structure that is configured to move the respective magnet relative to the common base structure in a lateral direction (or two lateral directions) perpendicular to the beam path. For example, the focusing assembly may comprise a second translation stage to which the downstream quadrupole magnet is mounted. The second translation stage may be configured to move the downstream quadrupole magnet laterally with respect to the beam path for scanning the target point perpendicular to the beam path. The second translation stage may be a two-axis translation stage (e.g., a pair of linear translation stages) that is configured to move the downstream quadrupole magnet in two lateral directions (e.g., both horizontally and vertically) for scanning the target point in two directions perpendicular to the beam path. This may for example allow for performing pencil beam scanning of the ion beam (e.g., across a target or target area). Using the downstream quadrupole magnet for lateral scanning may be advantageous since the ion beam may have a smaller size at the downstream quadrupole magnet than at the upstream and central quadrupole magnets and/or the downstream quadrupole magnet may be fixed along the beam path (i.e., may not be moveable longitudinally).

In some examples, the focusing assembly may further comprise a third translation stage to which the central quadrupole magnet is mounted. The third translation stage may be mounted to the first translation stage and may be configured to move the central quadrupole magnet longitudinally along the beam path relative to the upstream quadrupole magnet for adjusting the central drift length (e.g., for adjusting the central drift length and the downstream drift length simultaneously). The third translation stage may for example be used for minor adjustments, e.g., to correct for minor deviations from ideal quadrupole behavior, while using the first translation stage common to the upstream and central quadrupole magnets for (coarse) adjustment of the focus point. In other examples, the upstream quadrupole magnet and the central quadrupole magnet may each be mounted individually to a respective translation stage, e.g., the upstream quadrupole magnet to a first translation stage and the central quadrupole magnet to a second translation stage (but not to the first translation stage).

Preferably, some or all of the quadrupole magnets are or comprise permanent magnets. The use of permanent magnets may for example be advantageous to reduce the physical dimensions and/or weight of the beamline, e.g., as compared to superconducting electromagnets, while still allowing for generating sufficiently large magnet field gradients (e.g., more than 100 T/m, preferably more than 200 T/m, in some examples more than 400 T/m). Some or all of the quadrupole magnets may be Halbach quadrupole magnets (e.g., comprise a plurality of magnetic segments arranged around a bore and exhibiting a spatially rotating magnetization pattern for generating a quadrupole field within the bore). The permanent magnets may for example be or comprise neodymium (NdFeB) magnets. Additionally or alternatively, the permanent magnets may be or comprise samarium-cobalt magnets, in particular Sm₂Co₁₇ magnets. As compared to neodymium magnets, samarium-cobalt magnets may have slightly smaller magnetic field strengths but may be more resistant to radiation and/or thermal effects.

The beamline may further comprise one or more radiation shielding plates arranged at an upstream entrance facet of a respective one of the quadrupole magnets. The radiation shielding plates may be configured to prevent ions from the ion beam from hitting the quadrupole magnets, e.g., to prevent radiation-induced demagnetization thereof. The radiation shielding plates may for example comprise or consist of a material having a high density and, optionally, a small nuclear charge Z (e.g., for stopping the ions) and/or a material having a large nuclear charge Z (e.g., for attenuating photons induced by the ions). The radiation shielding plates may for example comprise or consist of a metal such as brass or aluminum. Preferably, the radiation shielding plates comprise or consist of a material that only creates short-lived activation products when exposed to the ion beam, e.g., activation products having a half-life of less than 48 hours, in some examples less than 24 hours, preferably less than 12 hours, most preferably less than 6 hours, in one example less than 3 hours and in one example less than 1 hour. The radiation shielding plates may for example comprise or consist of graphite and/or high-density polyethylene.

The collimator may comprise a pair of apertures for collimating the ion beam, for example a first or upstream aperture (e.g., downstream of the energy degrader) and a second or downstream aperture downstream of the upstream aperture (and, e.g., upstream of the focusing assembly). The upstream aperture may for example serve to define a spatial extent (e.g., a diameter or width) of the ion beam, whereas the downstream aperture may for example serve to define a divergence (e.g., a maximum opening angle) of the ion beam. Each of the apertures may for example have an elliptical (e.g., circular) or polygonal shape (e.g., a square or rectangular shape). Preferably, one or both of the upstream and downstream apertures are adjustable apertures having an adjustable aperture size (e.g., an adjustable diameter or width), for example to adjust the spatial extent and/or divergence of the ion beam. The apertures may for example comprise or consist of a material with a high density and, optionally, a small nuclear charge Z (e.g., for stopping the ions and/or causing less activation, neutron production and lateral scattering). The apertures may for example comprise or consist of a metal such as brass, tungsten, lead and/or a metal alloy (e.g., a bismuth alloy such as Wood's metal/Cerrobend). Preferably, the aperture comprises or consists of graphite. As compared to brass, for example, graphite may exhibit less long-term activation when exposed to ions from ion beam.

In some examples, one or both of the upstream and downstream apertures of the collimator may each be formed by one or more slits (extending in a lateral direction), in particular by a pair of orthogonal slits (e.g., one slit extending along a first lateral direction such as the X direction and one slit extending along a second lateral direction perpendicular to the first lateral direction such as the Y direction, thus in conjunction defining a square or rectangular aperture). The slits may be arranged adjacent to each other along the beam path. One or both of the apertures may each be formed by one or more pairs of collimator plates, wherein each pair may for example define a respective slit (i.e., the collimator plates of a given pair may be separated by a slit). One or both of the apertures may in particular be formed by two pairs of collimator plates that are rotated by 90° with respect to each other (e.g., such that one of the pairs defines a slit extending along the X direction and the other pair defines a slit extending along the Y direction). The collimator plates within each pair may be configured to move by a same distance in opposite directions for adjusting a size of the respective aperture (e.g., a width in the X or Y direction). This may ensure that the center of the aperture does not change when adjusting the size of the respective aperture (e.g., remains aligned with the beam path and/or the magnetic axes of the quadrupole magnets). The collimator plates within each pair may for example be coupled mechanically so as to move by the same distance in opposite direction. In particular, for some or all of the pairs of collimator plates, the collimator plates of the respective pair may be mounted to translation stage slides on an actuated double threaded spindle for synchronously moving the collimator plates in opposite directions (by rotation of the double threaded spindle).

In some examples, the collimator may further comprise an elongated collimation tube (e.g., a cylindrical or rectangular tube) that is arranged between the pair of apertures (e.g., extends between the pair of apertures). The elongated collimation tube may for example be arranged so as to block stray radiation originating from the upstream aperture (e.g., due to large-angle scattering at the upstream aperture). The elongated collimation tube may for example comprise or consist of graphite. Additionally or alternatively, the elongated collimation tube may also comprise or consist of any of the other materials listed above for the apertures of the collimator or a combination thereof.

In some examples, the beamline may comprise one or more beam shaping apertures that are arranged between a respective pair of adjacent quadruple magnets along the beam path. The beam shaping apertures may be configured to limit an extent of the ion beam at least in a direction in which the downstream one (i.e., the more downstream magnet) of the respective pair of quadrupole magnets is defocusing. The beam shaping apertures may for example be embodied as a slit extending in the direction in which the subsequent quadrupole magnet is focusing or as a circular or polygonal (e.g., rectangular) aperture. The beamline may in particular comprise (at least) a shaping aperture that is arranged between the downstream-most quadrupole magnet along the beam path (e.g., the downstream quadrupole magnet of a quadrupole triplet) and the closest quadrupole magnet upstream thereof (e.g., the central quadrupole magnet of a quadrupole triplet) along the beam path. Preferably, the beam shaping aperture is configured to limit an extent of the ion beam at least in a direction in which the downstream-most quadrupole magnet is defocusing.

In some examples, the beamline may comprise a housing (e.g., an enclosure, case or box) in which the energy degrader and/or the common base structure with the collimator and the focusing assembly are arranged. The housing may for example comprise or consist of a metal such as aluminum and/or steel and/or acrylic glass. The housing may be configured to one or both of hold helium at atmospheric pressure and hold a vacuum of less than 1 mbar, preferably less than 0.5 mbar, most preferably of less than 0.1 mbar, in one example of less than 0.05 mbar and in one example of less than 0.01 mbar. As compared to ambient air, a helium atmosphere or vacuum may for example reduce scattering of the ion beam and may thus for example result in higher transmission, lower energy loss and/or smaller beam size.

The housing may comprise an upstream entrance window (e.g., defining an entrance aperture of the beamline through which the ion beam enters the beamline) and a downstream exit window (e.g., defining an exit aperture of the beamline through which the ion beam leaves the beamline). The entrance and exit windows may for example be formed of a polyimide and/or polyethylene terephthalate film. The polyimide and/or polyethylene terephthalate film may have a thickness of less than 500 µm, preferably of less than 200 µm, more preferably of less than 100 µm, most preferably of less than 20 µm and in one example of less than 15 µm. Thinner films may result in less scattering at the respective window such that the film thickness is preferably chosen as low as possible (but, e.g., still sufficiently thick for holding helium and/or a vacuum). The use of helium may be advantageous since it may allow for reducing the film thickness further than for vacuum.

In some examples, the beamline may comprise a monitoring chamber, in particular an ionization-based monitoring chamber (e.g., an ionization chamber), for monitoring the ion beam. The monitoring chamber may share the entrance window or preferably the exit window with the housing (i.e., the entrance or exit window of the housing may at the same time form the exit and entrance window, respectively, of the monitoring chamber, thus avoiding the need for a separate window).

The beamline may be mounted or mountable to a supporting platform such as a table, in particular an optical table. Preferably, the supporting platform is an adjustable supporting platform, in particular an adjustable optical table, that is configured to adjust a position and/or an orientation of the beamline (with respect to the ion beam source) for aligning the beam path of the beamline to the ion beam of the ion beam source. The supporting platform may be height-adjustable (e.g., have height-adjustable feet) for moving the beamline along the Y/vertical direction and/or rotating the beamline about the X and/or Z directions. Additionally or alternatively, the supporting platform may be moveable in the X and/or Z directions for moving the beamline along the X and/or Z directions and/or rotating the beamline about the Y/vertical direction. The supporting platform may for example be mounted on casters and/or may comprise an upper part for mounting the beamline thereon (e.g., a table top or plate) and a lower part (e.g., a frame or base) supporting the upper part, wherein the upper part may be moveable (e.g., slidable) with respect to the lower part in the X and/or Z directions.

In some examples, the common base structure maybe mounted moveably in the beamline for moving the collimator and the focusing assembly into and out of the beam path while preserving relative alignment between the collimator and the two or more quadrupole magnets. The common base structure may for example be moveable between a park (or disengaged or imaging) position (e.g., for broad beam irradiation mode as described below), in which the collimator and the focusing assembly are not arranged in the beam path (i.e., such that the ion beam does not pass through the collimator or the quadrupole magnets), and an engaged position (e.g., for focused irradiation mode as described below), in which the collimator and the focusing assembly are arranged in the beam path (i.e., such that the ion beam passes through the collimator and the quadrupole magnets). The common base structure may for example be mounted moveably on a guiding structure such as one or more rails.

The beamline may be configured to switch (e.g., manually and/or remotely or automatically) between a focused irradiation mode and one or more secondary irradiation modes (such as a broad beam irradiation mode). In the focused irradiation mode, the collimator and the focusing assembly are arranged in the beam path (e.g., by moving the common base structure into the engaged position and/or by placing the quadrupole magnets in the kinematic mounts) and, if present, a scattering foil is not arranged in the beam path. In the secondary irradiation mode(s), the (entire) focusing assembly or one or more parts thereof (e.g., one or more of the quadrupole magnets) and, optionally, the collimator are not arranged in the beam path (e.g., by moving the common base structure into the park position and/or removing the quadrupole magnets from the beamline/the kinematic mounts). For example, in the broad beam irradiation mode, the (entire) focusing assembly and, optionally, the collimator may not be arranged in the beam path (e.g., by moving the common base structure into the park position and/or removing all of the quadrupole magnets from the beamline/the kinematic mounts)If present, a scattering foil may be arranged in the beam path in one or more of the secondary irradiation modes (e.g., the broad beam irradiation mode). As another example of a secondary irradiation mode, one of the quadrupole magnets may be removed to form a doublet focusing assembly (comprising the two other quadrupole magnets still arranged in the beam path), for example to deliver an elliptical (e.g., oval shaped) parallel beam (e.g., with relatively higher intensity, for example for FLASH beam delivery) Thereby, the beamline may provide either a focused ion beam or a non-focused (or differently focused) ion beam. This may allow for implementing various irradiation modes or schemes with the beamline with only minimal modifications, for example for ion imaging, passively scattered beam delivery, FLASH beam delivery and/or minibeam irradiation.

For example, the beamline may further comprise an additional collimator for imaging and/or passively collimated beam delivery (e.g., for passively scattered beam delivery, FLASH beam delivery and/or minibeam irradiation). The additional collimator maybe arranged closer to the target point than the (main) collimator on the common base structure, e.g., downstream of the quadrupole magnets, e.g., in front of or within the exit aperture or window or even downstream of the exit aperture or window (e.g., adjacent to or within a target holder). In some examples, the additional collimator may comprise a plurality of apertures (e.g., of different sizes and/or shapes) that are displaced laterally with respect to each other (e.g., in a plane perpendicular to the beam path). The beamline may be configured to move the additional collimator for selectively moving a respective one of the plurality of apertures into the beam path. In a preferred embodiment, the additional collimator is or comprises a multi-slit collimator for minibeam irradiation, e.g., as described in C. Guardiola et al., Medical Physics, Vol. 44, Issue 4 April 2017, pp. 1470-1478 or F. Reaz et al., Scientific Reports (2024) 14:8468*.* Additionally or alternatively, the additional collimator may be or comprise a compensator and/or 3D modulator for passively scattered beam delivery and/or FLASH beam delivery, e.g., as described in F. Horst et al., Frontiers in Physics, Vol. 11 (2023), DOI 10.3389/fphy.2023.1213779*.* In some examples, the additional collimator may comprise a target-specific aperture, e.g., an aperture shaped according to a shape of a target such as a tumor.

In some examples, the beamline may further comprise a scattering foil that is arrangeable in the beam path for the ion beam to pass therethrough. Additionally or alternative, the energy degrader (e.g., one or more degrading members thereof) may be used as a scattering element for scattering the ion beam. In focused irradiation mode, the scattering foil may not be arranged in the beam path, whereas in broad beam irradiation mode the scattering foil may be arranged in the beam path. The scattering foil may for example be part of or arranged adjacent to the energy degrader. The scattering foil may be mechanically coupled to the degrader (e.g., to a degrading member thereof), for example such that the degrader (e.g., the respective degrading member thereof) may be moved out of beam path and the scattering foil into the beam path and vice-versa. The scattering foil may for example comprise or consist of tantalum, lead, plastic and/or aluminum. The scattering foil may for example comprise or consist of a material with a mixture of elements of both low and high nuclear charge (Z), e.g., to have a differential degree of scattering).

The beamline according to the present invention may be modified in various ways. For example, the quadrupole magnets of the focusing assembly may be mounted differently, e.g., in a non-removable fashion (e.g., mounted permanently via kinematic coupling) and/or using a different mount (e.g., a non-kinematic mount). Additionally or alternatively, the common base structure may be omitted in some examples. The collimator and the focusing assembly may for example be mounted to separate base structures instead or directly on the supporting platform (with, optionally, translation stage(s) therebetween). Irrespective of the mounting of the quadrupole magnets, the focusing assembly and the collimator, the beamline may comprise any combination of the other features disclosed herein, in particular the translation stages as described above.

The components of the beamline, in particular the collimator, the focusing assembly, and/or a kinematic mount for a quadrupole magnet, may also be provided as separate components (optionally, in combination with the respective stage(s) for example) independently of the beamline and the other components.

The present invention further provides a method of aligning a transportable beamline according to any one of the embodiments disclosed herein. The method comprises 1) mounting the collimator and the focusing assembly to the common base structure, 2) for each of the two or more quadrupole magnets, aligning a magnetic axis of the quadrupole magnet to the beam path for the ion beam by adjusting a position and/or an orientation of the quadrupole magnet relative to the common base structure, and 3) fixing the position and the orientation of each of the two or more quadruple magnets relative to the common base structure. The above numbering is for illustrative purposes only and not intended to limit the method to any particular order of execution. As far as technically feasible, the method may be executed in an arbitrary order and steps thereof may also be executed simultaneously at least in part.

The method may be used for an initial alignment of the beamline (e.g., prior to first use, prior to transport to an ion beam source facility and/or prior to first installation at the ion beam source facility). Subsequently, the internal alignment of the beamline may be preserved by the common base structure such that no further internal alignment may be needed (e.g., when re-installing the beamline at the same facility and/or transporting the beamline to another facility). The method may, however, also be used for later re-alignment of the beamline (e.g., after transport to a new facility).

The collimator (e.g., the apertures thereof) and the focusing assembly (e.g., the quadruple magnets thereof) may be mounted to the common base structure directly and/or indirectly (in particular with one or more translation stages arranged therebetween). Mounting the focusing assembly to the common base structure may comprise mounting the two or more quadrupole magnets of the focusing assembly to the common base structure via a respective mount, in particular via their respective kinematic mount. This may comprise coupling a mount portion of the respective mount to the common base structure (e.g., attaching the mount portion to the common base structure or an element attached thereto such as a translation stage). Additionally or alternatively, this may comprise arranging the respective quadrupole magnet in a holder portion of the respective mount and/or coupling (in particular kinematically coupling) the holder portion to the mount portion (e.g., placing the holder portion on the mount portion or an intermediate portion arranged on the mount portion).

The alignment of the magnetic axes of the quadrupole magnets to the beam path may be performed prior to and/or after mounting the respective quadrupole magnet to the common base structure. Accordingly, the position and/or orientation of the quadrupole magnet relative to the common base structure may be adjusted once the quadrupole magnet is mounted to the common base structure and/or before mounting the quadrupole magnet to the common base structure (i.e., by adjusting a position and/or orientation in which the quadrupole magnet is to be mounted relative to the common base structure, e.g., by adjusting a mount (or a part or portion thereof) for the quadrupole magnet prior to arranging the magnet therein). The magnetic axes of the quadrupole magnets may be aligned so as to be parallel to the beam path, in particular so as to coincide with the beam path (e.g., a corresponding portion thereof extending through the quadrupole magnet). The magnetic axes of the quadruple magnets may for example be aligned so as to coincide with the beam path (e.g., as defined by the collimator) with a tolerance of no more than 100 µm, preferably no more than 50 µm, most preferably no more than 30 µm, in one example no more than 20 µm and in one example no more than 15 µm.

The position and the orientation of the quadrupole magnets relative to the common base structure may be fixed actively, e.g., using one or more locking means such as screws and/or clamps, and/or passively, e.g., by mounts configured to maintain the position and the orientation of the respective quadrupole magnet (for example by friction and/or gravity) unless being actively adjusted or moved.

Some or all of the two or more quadrupole magnets may be mounted to the common base structure via a respective adjustable kinematic mount, e.g., as detailed above for the beamline according to the present invention. The adjustable kinematic mounts may each comprise a set of adjustable mount points, e.g., the first set of mount points described above (which may for example couple the mount portion and the intermediate portion and/or may be formed by a set of height-adjustable pins and/or formed and/or supported by an upper part of the mount portion that is slidably arranged on a bottom part of the mount portion). The magnetic axes of the respective quadrupole magnets may be aligned to the beam path for the ion beam by (e.g., among other) adjusting at least a first mount point of the set of mount points (in some examples at least two or all of the set of mount points) of the respective adjustable kinematic mount.

In some examples, aligning the magnetic axes of said two or more quadrupole magnets may comprise: in a step a), for each of the two or more quadrupole magnets, adjusting the first mount point of the respective adjustable kinematic mount to a nominal height above the common base structure along a first lateral direction perpendicular to the beam path; and subsequently in a step b), for each of the two or more quadrupole magnets, adjusting the remaining mount points of the set of mount points of the respective adjustable kinematic mount to the nominal height above the common base structure. The mount points may for example be adjusted to the nominal height by adjusting the height of the corresponding height-adjustable pin. The nominal height may, e.g., be the height of the mount points when the magnetic axis of the respective quadrupole magnet is aligned with the beam path. The first lateral direction may correspond to the first direction as defined above, e.g., the Y/vertical direction.

Additionally or alternatively, aligning the magnetic axes of said two or more quadrupole magnets may comprise: in a step c), for a first one and a second one of said two or more quadrupole magnets, adjusting the first mount point of the respective adjustable kinematic mount to its nominal position along the beam path and a second lateral direction, the second lateral direction being perpendicular to the beam path and to the first lateral direction; and subsequently in a step d), for each of the two or more quadrupole magnets, adjusting the remaining mount points (and, optionally, also the first mount point again) of the respective adjustable kinematic mount to their nominal positions along the beam path and the second lateral direction. The mount points may for example be adjusted to their nominal position by sliding the upper part of the mount portion (which forms and/or supports the mount points) relative to the bottom part of the mount portion. The nominal position of a mount point may, e.g., be the position of the mount point when the magnetic axis of the respective quadrupole magnet is aligned with the beam path. The second lateral direction may correspond to the second direction as defined above, e.g., the X/horizontal direction. Preferably, the first quadrupole magnet is the upstream-most quadrupole magnet along the beam path of the quadrupole magnets of the focusing assembly (e.g., the upstream quadrupole magnet of a quadrupole triplet) and/or the second quadrupole magnet is the downstream-most quadrupole magnet along the beam path of the quadrupole magnets of the focusing assembly (e.g., the downstream quadrupole magnet of a quadrupole triplet).

Steps a) and c) may be performed prior to performing steps b) and d). Steps a) and c) may be performed simultaneously at least in part (e.g., using a mechanical alignment template). Step b) may be performed prior to performing step d) or may be performed simultaneously with step d) at least in part.

In a preferred embodiment, the mount points of the adjustable kinematic mounts are adjusted using a mechanical alignment template, e.g., as described above. The alignment template may have a plurality of precision holes and/or recesses arranged in a pattern corresponding to the nominal positions (e.g., along the beam path and the second lateral direction) of at least a subset (e.g., one, two or all) of the mount points for each of the adjustable kinematic mounts. Each of the precision holes and/or recesses may be configured to receive a respective one of the mount points of the adjustable kinematic mounts for mechanical alignment of the mount points of the adjustable kinematic mounts with respect to each other. The alignment template may be used for one or both of relative adjustment/alignment of the mount points with respect to each other and/or absolute adjustment/alignment of the mount points with respect to the beam path. In some examples, the method may comprise aligning the alignment template relative to the beam path (e.g., relative to entrance and exit apertures of the beamline), for example using an alignment laser beam.

In some examples, some or all of the adjustable kinematic mounts may each comprise a mount portion for coupling to the common base structure, a holder portion holding the respective quadrupole magnet and an intermediate portion arranged between the mount and holder portions, e.g., as described above. For each of said some or all of the adjustable kinematic mounts, the respective adjustable kinematic mount may comprise a second set of mount points coupling the intermediate portion and the holder portion (e.g., in addition to the first set of mount points coupling the mount portion and the intermediate portion of the respective adjustable kinematic mount), for example as described above.

The method may further comprise, for at least one, preferably for all of said some or all of the adjustable kinematic mounts, adjusting one or more (in some examples all) mount points of the second set of mount points of the respective adjustable kinematic mount to align the magnetic axis of the respective quadrupole magnet relative to the intermediate portion of the adjustable kinematic mount and subsequently fixing the position and the orientation of the quadruple magnet relative to the intermediate portion (e.g., using a locking means as described above). This may comprise determining the position and/or orientation of the magnetic axis of the respective quadrupole magnet, e.g., using a stretched wire, precision Hall-probe and/or rotating coil method. The magnetic axis of the respective quadrupole magnet may for example be aligned to a pre-defined reference position and/or orientation relative to the intermediate portion, e.g., such that the position and/or orientation of the quadrupole magnet relative to the intermediate portion is known (and fixed) and need only be determined once. The mounts points of the second set of mount points may for example be adjusted by tilting and/or translating a tip/tilt plate of the intermediate portion (that forms and/or supports the second set of mount points) with respect to a base of the intermediate portion, e.g., as described above.

The method may further comprise aligning the collimator relative to the magnetic axes of said two or more quadrupole magnets, e.g., such that the collimator (e.g., the apertures of the collimator and in particular the centers thereof) are aligned with the beam path and/or with the magnetic axes (i.e., lie on the same line as the magnetic axes). The collimator may be aligned using an optical telescope. The collimator may for example be aligned relative to the magnetic axes of the quadrupole magnets by aligning an optical axis of an optical telescope to the magnetic axes of the two or more quadrupole magnets (e.g., using optical reference markers of the alignment template and/or on the quadrupole magnets or their mounts) and aligning the collimator to the optical axis of the telescope.

In some examples, the method may comprise mounting the beamline to a supporting platform, in particular an optical table, e.g., as described above. The method may further comprise adjusting a position and/or an orientation of the supporting platform (e.g., prior to and/or after mounting the beamline thereto) to align the beam path of the beamline to an ion beam of an ion beam source, for example to align an alignment laser beam of the ion beam source to the entrance and exit apertures of the beamline.

### LIST OF FIGURES

In the following, a detailed description of the invention and exemplary embodiments thereof is given with reference to the figures. The figures show schematic illustrations of
Fig. 1: a transportable beamline for ion beam irradiation according to an example in a cross-sectional side view;
Fig. 2: a transportable beamline for ion beam irradiation according to another example in a cross-sectional side view;
Fig. 3A, 3B: an adjustable kinematic mount for removably mounting a quadrupole magnet in a transportable beamline according to an example in exploded perspective views;
Fig. 4: an adjustable collimator slit for use in a transportable beamline according to an example in a perspective view;
Fig. 5: an adjustable degrader for use in a transportable beamline according to an example in a perspective view;
Fig. 6: an adjustable supporting platform for a transportable beamline according to an example in an exploded perspective view;
Fig. 7: a flow diagram of a method of aligning a transportable beamline according to an example;
Fig. 8A, 8B: a mechanical alignment template for aligning a transportable beamline according to an example in a perspective view and bottom view, respectively; and
Fig. 9: a flow diagram of a method of irradiating a target point using a transportable beamline according to an example.

### DESCRIPTION OF THE EXEMPLARY EMBODIMENTS

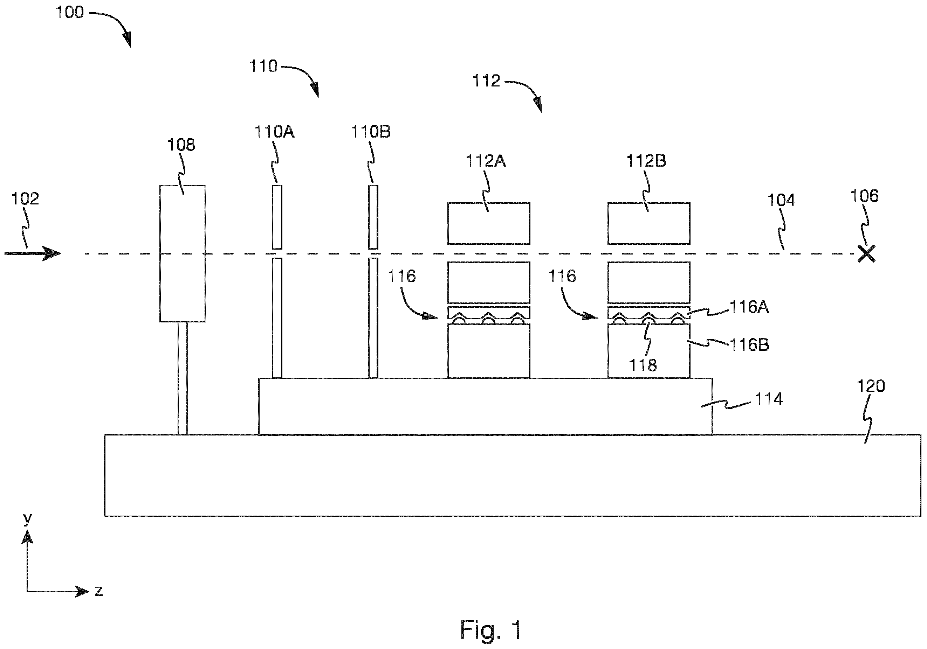

Fig. 1 depicts a schematic illustration (not to scale) of a transportable beamline 100 for ion beam irradiation according to an example in a cross-sectional side view. The beamline 100 is configured to receive an ion beam 102 such as a proton beam from an ion beam source (not shown). The beamline 100 is configured to guide the ion beam 102 along a beam path 104 to a target point 106, at which a target (not shown) may be arranged (e.g., in a suitable target holder). In the example of Fig. 1, the beam path 104 extends along the Z direction (longitudinal direction).

In one example, the ion beam source is an ion beam source at a clinical facility for particle therapy. The ions in the received ion beam 102 may thus for example have an initial energy between 50 MeV/mᵤ and 250 MeV/mᵤ, in some examples between 50 MeV/mᵤ and 100 MeV/mᵤ, in one example between 70 MeV/mᵤ and 100 MeV/mᵤ (with mᵤ denoting the mass of the ions in atomic mass units, i.e., mᵤ = 1 for protons) and a lateral extent (e.g., a diameter or spot size) of the received ion beam 102 (e.g., at a focus point of the ion beam source) may for example be between 2 mm and 6 mm, in one example between 3 mm and 5 mm. The beamline 100 may be configured to derive an ion beam suitable for small animal ion beam irradiation from the received ion beam 102 (e.g., for a mouse as the target). The energy of the ions and lateral extent of the received ion beam 102 from the ion beam source, which may be designed for the irradiation of human subject or patient, may be too large for this purpose, which may for example require an ion beam with ions having an energy between 10 MeV/mᵤ and 60 MeV/mᵤ, preferably between 20 MeV/mᵤ and 50 MeV/mᵤ, and/or a lateral extent of the ion beam of between 0.2 mm and 2.0 mm, in one example between 0.5 mm and 1.5 mm. The beamline 100 may thus be configured to manipulate the received ion beam 102 accordingly so as to provide such an ion beam suitable for small animal ion beam irradiation at the target point 106. In other examples, the beamline 100 may be configured for other purposes, e.g., configured to receive an ion beam 102 having a different initial energy and/or a different lateral extent and/or to derive an ion beam having a different target energy and/or a different lateral extent.

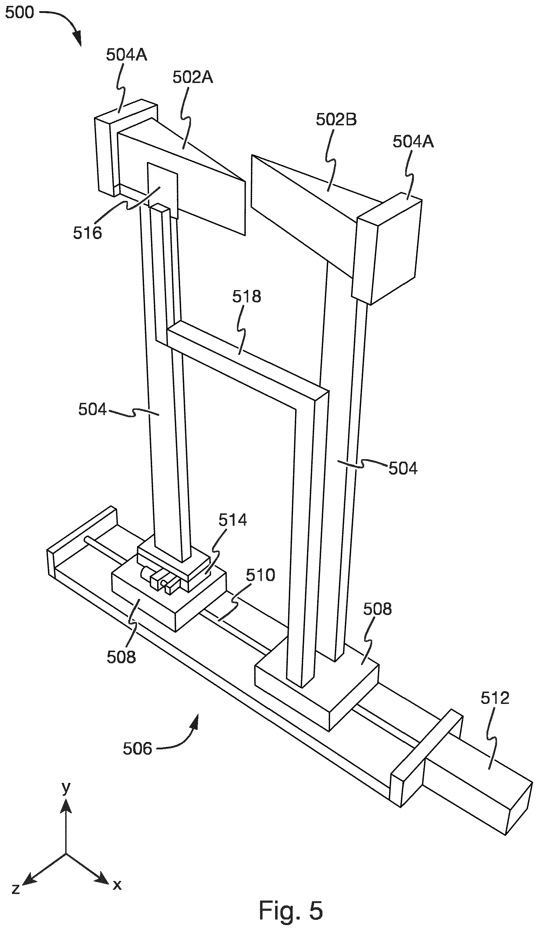

The beamline 100 comprises an energy degrader 108 that is configured to decelerate the ions in the ion beam 102 to a target energy in the desired range. The energy degrader 108 comprises a degrading member that is arranged (or arrangeable) in the beam path 104. The degrading member comprises a piece of material (e.g., a slab, plate or wedge of material) through which the ion beam 102 passes such that the ions in the ion beam 102 are decelerated by interaction with the material. The amount of deceleration may depend on the thickness of the material through which the ion beam 102 passes. The target energy to which the ions are decelerated may thus be adjusted by changing the thickness of the material, e.g., using another degrading member of different thickness or using an adjustable degrader having a degrading member of varying thickness, e.g., as described below with reference to Fig. 5. The material of the degrading member is not particularly limited and may for example be or comprise boron carbide (e.g., for reducing emittance growth of the degraded beam in the energy degrader). In some examples, the energy degrader 108 is embodied similar to the energy degrader 108 of Fig. 5.

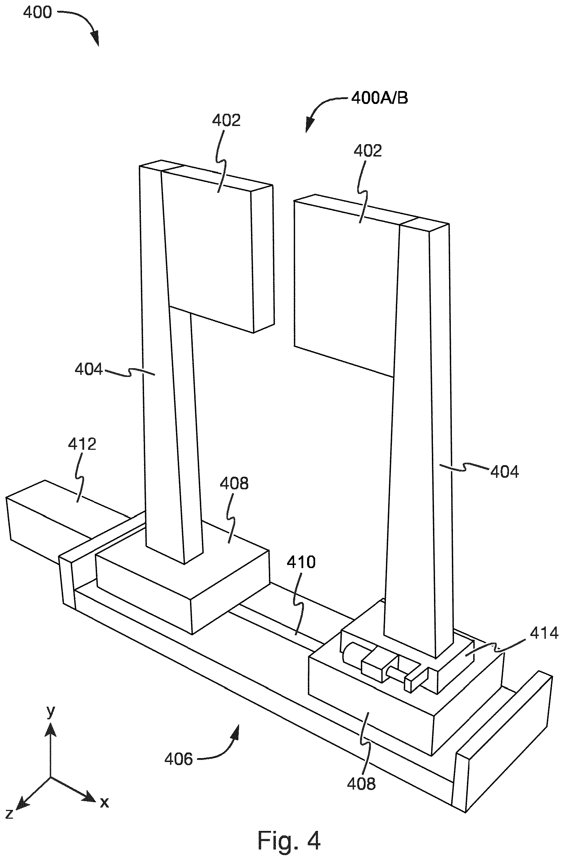

The beamline 100 further comprises a collimator 110 (which may also be referred to as a collimator assembly) that is arranged downstream of the energy degrader 108. The collimator 110 is configured to collimate the degraded ion beam 102 and for this comprises a pair of apertures 110A, 110B with the upstream aperture 110A defining the lateral extent (e.g., diameter) of the ion beam 102 and the downstream aperture 110B defining the divergence (e.g., opening angle) of the ion beam 102. The apertures 110A, 110B may for example be elliptical (e.g., circular) or rectangular (e.g., square) apertures. Each of the apertures 110A, 110B may for example comprise a pair of orthogonal slits together defining a rectangular or square opening, e.g., as detailed below with reference to Fig. 4. The material of the apertures 110A, 110B is not particularly limited and may for example be or comprise graphite (e.g., to avoid long-term activation). In some examples, the collimator 110 is embodied similar to the collimator 400 of Fig. 4.

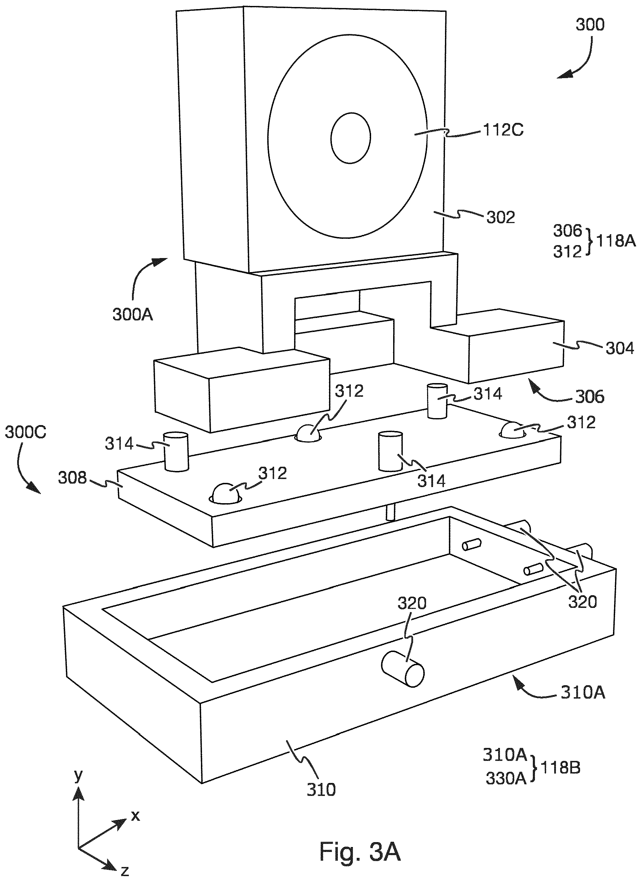

A focusing assembly 112 for focusing the ion beam 102 is arranged downstream of the collimator 110. The focusing assembly 112 comprises a pair of quadrupole magnets, namely an upstream quadrupole magnet 112A and a downstream quadrupole magnet 112B. The quadrupole magnets 112A, 112B are oriented such that the upstream quadrupole magnet 112A focuses the ion beam 102 in a first transverse plane (e.g., the Y-Z plane) and the downstream quadrupole magnet 112B focuses the ion beam 102 in a second transverse plane perpendicular to the first transverse plane (e.g., the X-Z plane). Put differently, the poles of the quadrupole magnets 112A, 112B are rotated by 90° with respect to each other (and, e.g., arranged at an angle of 45° with respect to the X and Y directions). In some example, one or both of the quadrupole magnets 112A, 112B are embodied as the quadrupole magnet of Fig. 3A.

The collimator 110 and the focusing assembly 112 (i.e., the quadrupole magnets 112A, 112B) are mounted to a common base structure 114. The base structure 114 provides a common reference frame or point of reference for the collimator 110 and the focusing assembly 112 so as to preserve the relative alignment between the collimator 110 and the quadrupole magnets 112A, 112B, e.g., during transport, installation and use of the beamline 100. The common base structure 114 is configured to prevent the collimator 110 and the quadrupole magnets 112A, 112B from moving relative to each other. The common base structure 114 may be configured to prevent (e.g., reduce or minimize) displacement between these components due to thermal drifts and/or external mechanical shocks (e.g., vibrations). For this, the common base structure 114 is formed as a single integral piece of material in the example of Fig. 1, e.g., a single integral block or slab of material. The material of the common base structure 114 may be chosen so as exhibit high mechanical stability, low thermal expansion, light weight and/or precision machinability (e.g., for screwing components thereto). The common base structure 114 may for example have a coefficient of linear thermal expansion of no more than 10 · 10⁻⁶/K. Suitable materials for the common base structure 114 include for example granite, a carbon fiber compound and/or an iron-nickel alloy (e.g., invar).

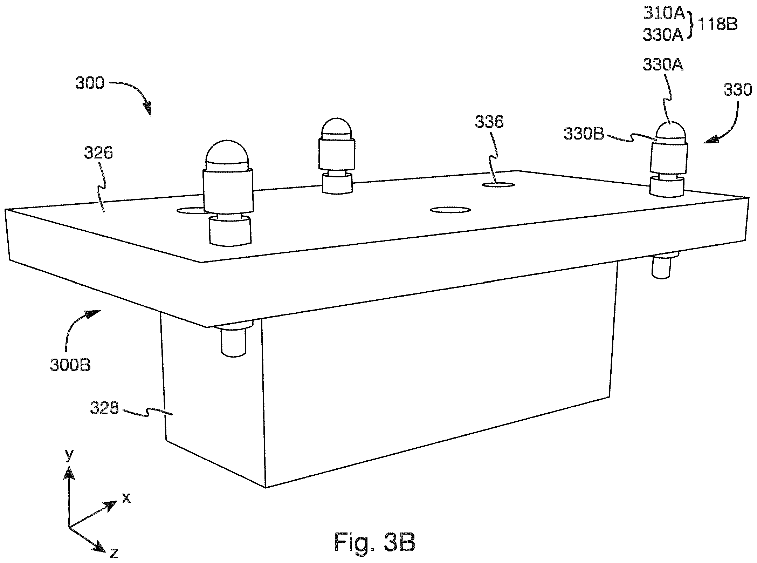

The quadrupole magnets 112A, 112B of the focusing assembly 112 are removably mounted to the common base structure 114 via a respective kinematic mount 116. In the example of Fig. 1, the kinematic mounts 116 each comprise a holder portion 116A holding the respective quadrupole magnet 112A/112B and a mount portion 116B that is coupled (e.g., mounted or attached) to the common base structure 114. The holder portion 116A and the mount portion 116B are kinematically coupled at a plurality (set) of kinematic mount points 118 (preferably at three kinematic mount points 118), e.g., such that the holder portion 116A is constrained in all six motional degrees of freedom but without over-constraining the holder portion 116A. The holder portion 116A may be removably arranged on the mount portion 116B, e.g., such that the holder portion 116A with the respective quadrupole magnet 112A/B may be removed from the mount portion 116B and placed thereon again (in some examples without requiring the use of tools, e.g., by taking or lifting off the holder portion from the mount portion 116B). In some examples, the kinematic mounts 116 may be adjustable kinematic mounts with moveable kinematic mount points 118, e.g., such that the position and the orientation of the respective quadrupole magnet with respect to the mount portion can be adjusted (preferably independently in all six degrees of freedom). The kinematic mount points 118 may for example be embodied similar to the (second set of) kinematic mount points of Fig. 3A and/or the (first set of) kinematic mount points of Fig. 3B.

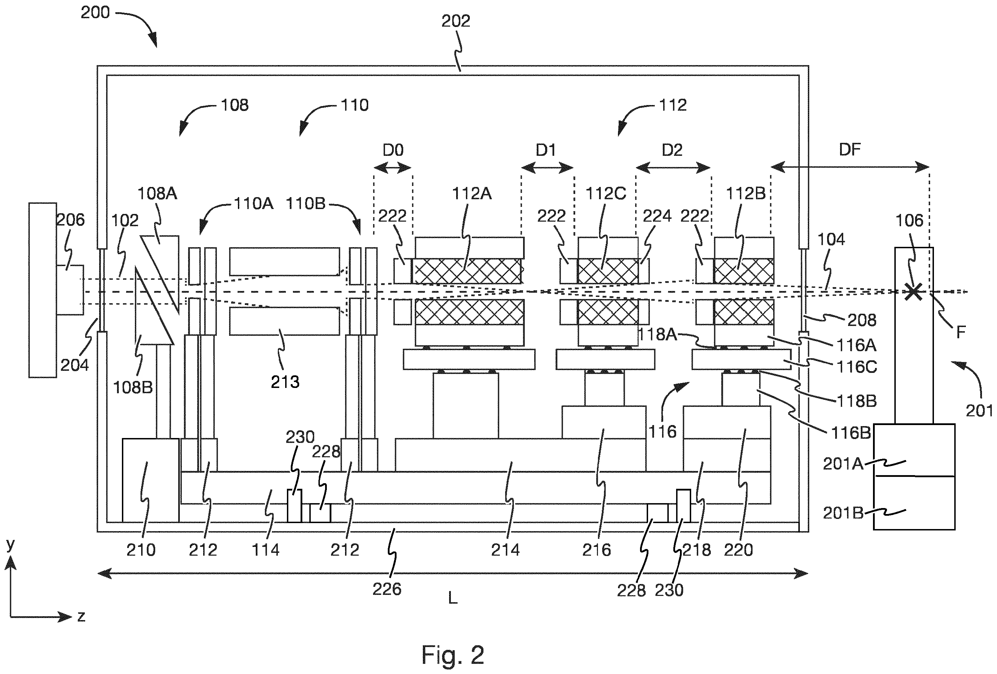

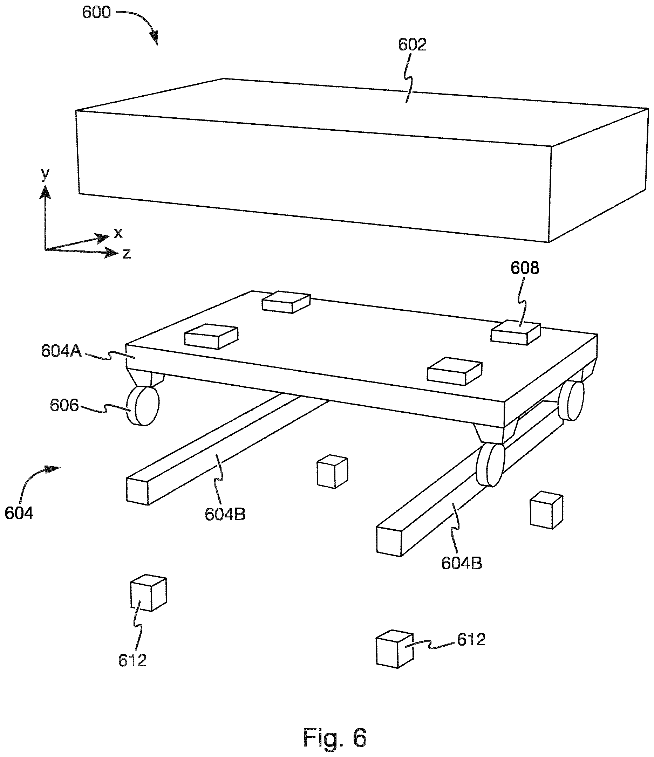

The common base structure 114 and the energy degrader 108 (whose alignment relative to the collimator 110 and the focusing assembly 112 is not as critical as the relative alignment between the collimator 110 and the quadrupole magnets 112A, 112B) are mounted to a supporting platform 120, for example an optical table. The supporting platform 120 may be adjustable (in position and/or orientation) and may for example be embodied similar to the supporting platform 600 of Fig. 6. In some examples, the energy degrader 108 may also be mounted to the common base structure 114 similar to the collimator 110 and the focusing assembly 112, e.g., as in the beamline 200 of Fig. 2.

Fig. 2 depicts a schematic illustration (not to scale) of a transportable beamline 200 for ion beam irradiation according to another example in a cross-sectional side view. The beamline 200 is similar to the beamline 100 of Fig. 1 and also comprises an energy degrader 108, a collimator 110 and a focusing assembly 112 to guide an ion beam 102 (illustrated in Fig. 2 by a pair of dotted lines indicating a lateral extent (e.g., 1/e² diameter or width) of the ion beam 102) received from an ion beam source (e.g., an outlet 206 thereof such as an exit aperture or window or a nozzle) along a beam path 104 (dashed line) to a target point 106.

The beamline 200 (and/or the beamline 100) may further comprise (or be configured for use with) a sample holder 201, which may for example be configured to hold (and preferably position) a sample or target such as a mouse (not shown) at the target point 106. In some examples the sample holder 201 may be configured to scan the sample or target in one or more directions (e.g., in addition to or instead of scanning the downstream quadrupole magnet 112B), for example at least in both transverse direction (X, Y) and, optionally, also in the longitudinal direction (Z). For this, the sample holder 201 may comprise (or be mounted to) one or more translation and/or rotation stages, for example a pair of (e.g., orthogonal) translation and/or rotation stages 201A, 201B as illustrated in Fig. 2. In some examples, a monitoring chamber and/or one or more additional collimators may be arranged between the focusing assembly 112 and the sample holder 201 and/or target point 106.

The energy degrader 108, the collimator 110 and the focusing assembly 112 are mounted to a common base structure 114 (e.g., a single piece of granite) and arranged in a housing 202, e.g., by mounting (e.g., attaching) the common base structure 114 to a base plate 226 of the housing 202. The housing 202 may for example be made of metal, e.g., aluminum or stainless steel. The housing 202 may be filled with helium (e.g., at atmospheric pressure or at a pressure of between 0.1 bar and 1 bar) or may contain a vacuum (e.g., at a pressure of between 10⁻³ mbar and 10⁻¹ mbar, e.g., 10⁻² mbar. A total length L of the beamline 200 (e.g., of the housing 202, for example from the entrance window 204 to the exit window 208) along the Z direction/beam path 104 may for example be between 0.4 m and 2 m, in some examples between 0.5 m and 1.2 m, in one example between 0.75 m and 1.0 m.

The housing 200 comprises an entrance aperture covered by an entrance window 204 facing the outlet 206 of the ion beam source. The housing 200 further comprises an exit aperture (or outlet) covered by an exit window 208 facing the target point 106. The entrance and exit windows 204, 208 may each be made of (e.g., comprise or consist of) a polyimide and/or polyethylene terephthalate film, e.g., a Kapton film. A respective thickness of the entrance and exit windows 204, 208 may for example be between 5 µm and 100 µm and may, e.g., be chosen to be as thin as possible for supporting the He filling or vacuum within the housing 202. In some examples, a monitoring chamber, e.g., an ionization chamber, may be arranged immediately downstream of the exit window 208, e.g., so as to share the exit window 208 with the housing 202 (as an entrance window of the monitoring chamber). The entrance and exit apertures/windows 204, 208 (e.g., the centers thereof) may define the beam path 104 or axis of the beamline 200, which in the example of Fig. 2 extends parallel to the Z direction.

The energy degrader 108 is an adjustable degrader configured to decelerate the ions in the ion beam 102 from their initial energy (which depending on the ion beam source may, e.g., be between 50 MeV/mᵤ and 100 MeV/mᵤ, in some examples between 70 MeV/mᵤ and 100 MeV/mᵤ (with mᵤ denoting the mass of the ions in atomic mass units u) and may be fixed or adjustable) to an adjustable target energy, for example adjustable across a range between 20 MeV/mᵤ and 50 MeV/mᵤ. For this, the energy degrader 108 comprises a pair of wedge-shaped degrading members 108A, 108B facing each other (e.g., in the y-direction) across the beam path 104 (such that a combined thickness of material that the ion beam 102 (e.g., the central and outer portions thereof) passes through is (at least substantially) independent of the lateral position in the transverse x-y-plane). At least one of the degrading members 108A, 108B is mounted moveably on an (e.g., linear) translation stage 210 configured to adjust a spacing between the degrading member 108A, 108B (e.g., along the y-direction). Thereby, an effective thickness/amount of material of the degrader 108 along the beam path 104 and thus the target energy (at a given initial energy) may be adjusted. In some examples, the energy degrader 108 may be embodied similar to the energy degrader 500 of Fig. 5.

The collimator 110 comprises a pair of apertures 110A, 110B for collimating the degraded ion beam 102. Each of the apertures 110A, 110B is formed by a pair of openings, e.g., a first/upstream slit extending along a first lateral direction (e.g., the x-direction/direction of view of Fig. 2) for collimating the ion beam 102 along the first lateral direction and a second/downstream slit extending along a second lateral direction (for example, perpendicular to the first lateral direction, e.g., along the y-direction) for collimating the ion beam 102 along the second lateral direction. One or both of the apertures 110A, 110B may be an adjustable aperture (e.g., an adjustable iris) whose aperture size may be varied, for example using a respective actuator or stage (e.g., translation stage) 212. In some examples, the openings of one or both of the apertures 110A, 110B may be embodied similar to the collimator slit 400A of the collimator 400 in Fig. 4. A spacing between the apertures 110A, 110B may for example be between 50 mm and 200 mm, in one example between 100 mm and 150 mm (e.g., 120 mm).

The collimator 110 further comprises an elongated collimation tube 213 that is arranged between the pair of apertures 110A, 110B. The elongated collimation tube 213 is configured to shield stray radiation originating from the upstream aperture 110A and may for example be made of (e.g., comprise or consist of) graphite (e.g., to avoid long-term activation). The elongated collimation tube 213 may for example be a cylindrical or rectangular tube. The elongated collimation tube may for example have a wall thickness between 10 mm and 100 mm, in some examples between 20 mm and 50 mm (e.g., 30 mm) and/or an inner diameter or width of between 4 mm and 20 mm (e.g., 10 mm). The elongated collimation tube 213 may for example extend over at least 50%, preferably over at least 75% of the spacing between the apertures 110A, 110B and/or may for example have a length between 50 mm and 200 mm, in one example between 80 mm and 120 mm (e.g., 100 mm).

The focusing assembly 112 is configured to focus the ion beam 102 at a focus point F, which may or may not coincide with the target point 106. For focusing the ion beam 102, the focusing assembly 112 comprises a triplet of three quadrupole magnets: an upstream quadrupole magnet 112A, a downstream quadrupole magnet 112B and a central quadrupole magnet 112C arranged between the upstream and downstream quadrupole magnets 112A, 112B. The upstream quadrupole magnet 112A is arranged at a spacing Do (the upstream drift length) downstream of the downstream aperture 110B of the collimator 110, the central quadrupole magnet 112C at a spacing D1 (the central drift length) downstream of the upstream quadrupole magnet 112A and the downstream quadrupole magnet 112B at a spacing D2 (the downstream drift length) downstream of the central quadrupole magnet 112C. A working distance of the beamline (which may for example be measured from the downstream facet of the downstream quadrupole magnet 112B or from the exit window 208 to the focus point F or to the target point 106) is denoted as DF and may for example be between 50 mm and 500 mm, in some examples between 100 mm and 300 mm, e.g., between 200 mm and 250 mm (for example to arrange additional elements for monitoring and/or imaging (e.g., an ionization chamber for monitoring) between the beamline 200 and the target and/or focus points 106, F). Accordingly, an effective length of the beamline (i.e., the distance from the entrance window 204 to the focus point F or to the target point 106, e.g., the sum of the length L and the working distance DF) may for example be between 0.5 m and 1.5 m, in one example between 0.9 m and 1.2 m.

The quadrupole magnets 112A-C each have an opening or bore (e.g., with an inner diameter or width between 10 mm and 30 mm, e.g., 20 mm) through which the ion beam 102 passes and are oriented so as to form a focusing-defocusing-focusing configuration for the ion beam 102 (i.e., focusing-defocusing-focusing in a first transverse plane, e.g., the y-z-plane and defocusing-focusing-defocusing in a second transverse plane perpendicular to the first transverse plane, e.g., the x-z-plane). The quadrupole magnets 112A-C may for example be permanent magnets, in particular samarium-cobalt magnets, and may, e.g., be embodied as Halbach quadrupole magnets,. The quadrupole magnets 112A-C may each be configured to generate an (e.g., equal) magnetic field gradient between 50 T/m and 500 T/m, in some examples between 100 T/m and 200 T/m (e.g., 160 T/m) within their respective bore. The quadrupole magnets 112A-C may form an asymmetric arrangement, e.g., have different lengths (along the beam path 104) and/or different magnetic field strengths/gradients. For example, the upstream quadrupole magnet 112A may have a shorter focal length (e.g., a greater length and/or a higher magnetic field strength) than the central and downstream quadrupole magnets 112C, 112B. In one example, the upstream quadrupole magnet 112A has a length of between 80 mm and 120 mm (e.g., 100 mm) and the central and downstream quadrupole magnets 112C, 112B have an (e.g., equal) length of between 30 mm and 70 mm (e.g., 50 mm).

Each of the quadrupole magnets 112A-C is removably mounted to the common base structure 114 via a respective kinematic mount 116. Each of the kinematic mounts 116 is a two-stage kinematic mount comprising a holder portion 116A holding the respective magnet 112A-C, a mount portion 116B attached to the common base structure 114 and an intermediate portion 116C arranged between the mount and holder portions 116B, 116A. The mount and intermediate portions 116B, 116C are kinematically (and, optionally, removably) coupled at a first plurality or set of kinematic mount points 118B, which preferably comprises or consists of three kinematic mount points 118B. The intermediate and holder portions 116C, 116A are kinematically (and, optionally, removably) coupled at a second plurality or set of kinematic mount points 118A, which preferably comprises or consists of three kinematic mount points 118A. The kinematic mount points 118A, 118B may uniquely define the position and orientation of the respective quadrupole magnet 112A-C relative to the common base structure 114 (in all six motional degrees of freedom) and may be configured to allow for (e.g., tool-less) removal and repositioning of the respective quadrupole magnet 112A-C from/ on the common base structure in precisely the same position and orientation. The quadrupole magnets 112A-C may thus be removed from the beamline 200 while maintaining their relative alignment with respect to each other and with respect to the collimator 110 (which effectively defines a reference point ("beam source" or "origin") of the ion beam 102 within the beamline 200 that is independent of the external ion beam source and the alignment of the beamline 200 relative to the ion beam source). Preferably, one or both of the first and second sets of kinematic mount points 118A, 118B are moveable to allow for adjusting a position and/or an orientation (preferably along all three directions/six motional degrees of freedom) of the intermediate portion 116C with respect to the mount portion 116B and of the holder portion 116A (with the respective quadrupole magnet 112A-C) with respect to the intermediate portion 116C, respectively. The first and/or second sets of mount points 118A, 118B and/or the holder, mount and/or intermediate portions 116A-C may for examples be embodied as for the kinematic mount 300 of Figs. 3A/B.

The focusing assembly 112 is configured to move the focus point F and/or the target point 106, e.g., along the beam path 104 and/or laterally in one or preferably two directions perpendicular to the beam path 104. The former may for example allow for adjusting the focus point F to the target energy (as the focal length of the quadrupole magnets 112A-C depends on the energy of the ions), e.g., to move or maintain the focus point F to/at the same position along the beam path 104 when changing the target energy (e.g., with the adjustable degrader 108). The latter may for example allow for scanning the target point 106 in the transverse plane perpendicular to the beam path 104 (e.g., to implement a pencil beam scanning irradiation). The focusing assembly 112 may be configured to move the focus point F along the beam path 104 by changing one or more of the drift lengths Do, D1, D2 and DF. The focusing assembly 112 may for example be configured to move the focus point F along the beam path 104 by changing the upstream drift length D₀ and the downstream drift length D2 (e.g., by equal amounts in opposite directions) while, optionally, maintaining the central drift length D1 and/or the working distance DF. Additionally or alternatively, the focusing assembly 112 may also be configured to move the focus point F along the beam path 104 by changing the magnetic field gradient of one or more of the quadrupole magnets 112A-C. For moving the target point 106 laterally, the focusing assembly 112 may for example be configured to move (e.g., shift or displace) one or more of the quadrupole magnets 112A-C laterally with respect to the beam path 104.

In the example of Fig. 2, the focusing assembly 112 comprises one or more translation stages 214 to 220, to which the quadrupole magnets 112A-C with their respective kinematic mount 116 are mounted (with the translation stages 214 to 220 (e.g., their bases or guiding structures) being mounted to the common base structure 114). Preferably, some or all of the translation stages (e.g., at least the first and second translation stages 214 and 218/220) are automated computer-controlled translation stages for moving the quadrupole magnets 112A-C remotely and, optionally, automatically without need for manual intervention within the beamline 200. Some or all of the translation stages 214 to 220 may be equipped with encoders for improved accuracy and/or repeatability.

In particular, the focusing assembly 112 comprises a first (e.g., linear) translation stage 214 that is common to both the upstream quadrupole magnet 112A and the central quadrupole magnet 112C (i.e., both of these magnets are mounted - via their respective kinematic mount - to the first translation stage 214). The first translation stage 214 is configured to simultaneously move the upstream and central quadrupole magnets 214 longitudinally along the beam path 104 (thus changing the drift lengths D₀ and D2 by equal, but opposite amounts while maintaining D1). This may be sufficient for moving the focus point F along the beam path 104 (e.g., for maintaining the focus point F at the same position along the beam path 104 for a range of target energies, e.g., at least between 20 MeV/mᵤ and 50 MeV/mᵤ) while only requiring a single translation stage/moving part. Optionally, the first translation stage 214 (and/or the third translation stage 216 described below) may also be configured to move the upstream and/or central quadrupole magnets 112A, 112C laterally with respect to the beam path 104 (e.g., be a two-axis or three-axis translation stage). In other examples, the upstream and/or central quadrupole magnets 112A, 112C may be fixed in the lateral directions.

The focusing assembly 112 further comprises a second translation stage 218/220, to which the downstream quadrupole magnet 112B - via its kinematic mount 116 - is mounted. The second translation stage 218/220 is configured to move the downstream quadrupole magnet 112B in one or preferably two lateral directions perpendicular to the beam path 102. The second translation stage 218/220 may for example be a linear (one-axis) translation stage, a two-axis translation stage or - as in the example of Fig. 2 - a pair of (e.g., stacked) linear translation stages 218 and 220 with different (e.g., orthogonal) orientations (for example one parallel to the y-direction and one parallel to the x-direction). In some examples, the downstream quadrupole magnet 112B may be fixed along the beam path 104.

In some examples, the focusing assembly 112 may further comprise a third (e.g., linear) translation stage 216, to which the central quadrupole magnet 112C - via its kinematic mount 116 - is mounted. The third translation stage 216 is in turn mounted to (and thus moveable by) the first translation stage 214. The third translation stage 216 is configured to move the central quadrupole magnet 112C longitudinally with respect to the upstream quadrupole magnet 112A for adjusting the central drift length D1 and the downstream drift length D2 (e.g., for correcting for deviations from ideal quadrupole behavior and/or optimizing a beam shape (e.g., aspect ratio) of the ion beam 102 at the target point 106 and/or focus point F).

In other examples, the focusing assembly 112 may comprise different arrangements of translation stages. For example, the upstream and central attachment magnets 112A, 112C may each be mounted individually to a respective translation stage (e.g., only the upstream magnet 112A to the first translation stage 214 and only the central magnet 112C to the second translation stage 216).

A radiation shielding plate 222 is arranged at the upstream entrance facet of each of the quadrupole magnets 112A-C. The radiation shielding plates 222 may be configured to shield the magnetic material of the respective quadrupole magnet 112A-C from the ion beam 102 and/or stray radiation. The radiation shielding plates 222 may for example be made of graphite.

A beam shaping aperture 224 is arranged upstream of the downstream quadrupole magnet 112B, e.g., at the downstream facet of the central quadrupole magnet 112C or somewhere else between the central and downstream quadrupole magnets 112C, 112B. The beam shaping aperture 224 is configured to limit a lateral extent of the ion beam at least in the direction in which the downstream quadrupole magnet 112B is defocusing (e.g., the x-direction) and may for example be embodied as a circular or rectangular aperture or as a slit extending along the direction in which the downstream quadrupole magnet 112B is focusing (e.g., the y-direction). The beam shaping aperture may be configured to prevent strongly diverging ions from the ion beam 102 and/or stray radiation from reaching the downstream quadrupole magnet 112B. The beam shaping aperture 224 may for example be made of graphite.

The collimator 110 and the quadrupole magnets 112A-C of the focusing assembly 112 are mounted to the common base structure 114, e.g., a block or table made of granite, a carbon fiber compound and/or an iron-nickel alloy such as invar. The common base structure 114 and the energy degrader 108 are mounted to a base plate 226, which as mentioned above may for example be the bottom wall of a housing such as the housing 202. The base plate 226 may be mounted to an adjustable supporting platform such as for the optical table 602 of the supporting platform 600 of Fig. 6 described below.

The common base structure 114 may be mounted so as to be moveable between a first position (also referred to as the "engaged position"), in which the collimator 110 and the quadrupole magnets 112A-C of the focusing assembly 112 are arranged in the beam path 104, and a second position (also referred to as the "disengaged position", "park position" or "imaging position"), in which the collimator 110 and the quadrupole magnets 112A-C of the focusing assembly 112 are not arranged in the beam path 104. The common base structure 114 may for example be mounted moveable on one or more guiding structures 228 (e.g., a pair of guiding structures as in Fig. 2) such as rails, linear guides or translation stages on the base plate 226. The common base structure 114 may be driven by one or more actuators (e.g., stepper motors). The beamline 200 may comprise one or more stops 230 (preferably adjustable in position) configured to limit the motion of the common base structure 114, in particular in the engaged position. For example, one stop may be provided for each of the guiding structure 228 (e.g., at an end thereof). The stops may be mechanical stops or preferably electrical stops (for example electrical contacts configured to stop some or all of the motors driving the common base structure when coming in contact with the common base structure.) This may for example prevent misalignment due to tolerance in the guiding structures and thus ensure an accurate position of the common base structure (e.g., in the engaged position).

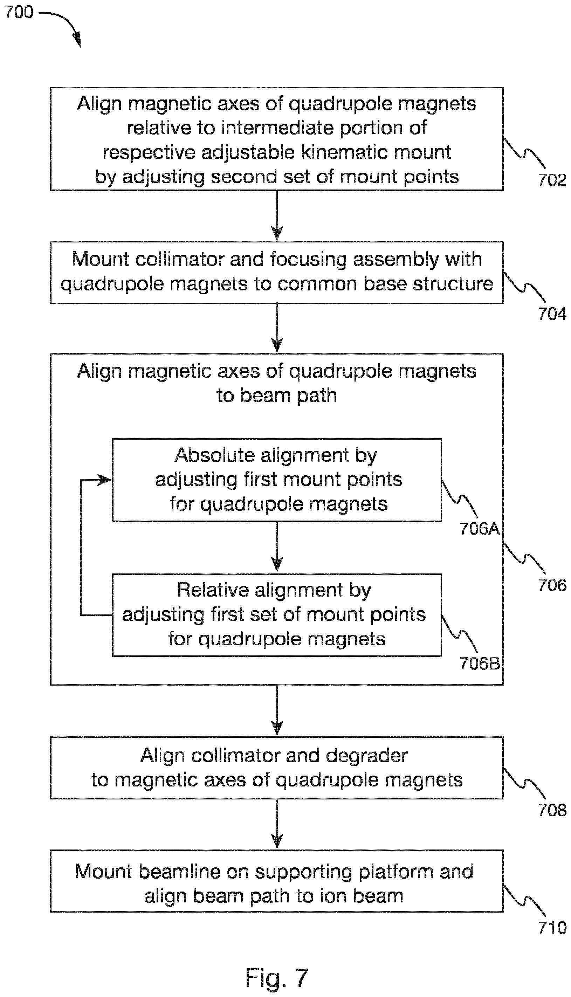

The moveable common base structure 114 in combination with the kinematic mounts 116 for the quadrupole magnets 112A-C (which allow for removal of the respective magnet, e.g., by taking off the holder portion 116A or the intermediate portion 116C) facilitate quick and simple transitions between various configurations for the beamline 200 (and/or for the beamline 100), for example for transport and/or implementing various irradiation modes, all while maintaining relative alignment between the pair of apertures 110A, 110B of the collimator 110 and the magnets 112A-C of the focusing assembly 112, thus avoiding or reducing the need for complex and time-consuming realignment thereof. Instead, the respective components may only be aligned once (e.g., prior to first use) or re-aligned infrequently (e.g., only after transport to a new ion beam source facility), for example using a method of aligning a transportable beamline according to any one of the examples described herein such as the method 700 described below with reference to Fig. 7.

For transport, the magnets 112A-C or the entire common base structure 114 with the collimator 110 and the focusing assembly 112 may for example be removed from the beamline 200 and subsequently be mounted therein again. Furthermore, the following irradiation modes may for example be implemented: 1) a focused irradiation mode with the common base structure 114 in the engaged position such that the collimator 110 and the focusing assembly 112 are arranged in the beam path 104, 2) an actively collimated/fan beam irradiation mode (as an example of a secondary irradiation mode) with the common base structure 114 in the engaged position such that the collimator 110 and all or some parts of the focusing assembly 112 are arranged in the beam path, 3) a broad beam (or non-focused) irradiation mode (as another example of a secondary irradiation mode) in which the focusing assembly 112 (or one or more parts thereof such as one or more of the quadrupole magnets 112A-C) is not arranged in the beam path, for example 3a) by removing the magnets 112A-C from the common base structure 114 (such that the collimator 110 remains in the beam path) or 3b) by moving the common base structure 114 to the park/disengaged position (such that the collimator 110 is also removed from the beam path). In either of these modes, a scattering foil such as the scattering foil 516 described below with reference to Fig. 5 may either be arranged in the beam path 104, e.g., in broad beam irradiation modes 3a) or 3b), or removed from the beam path 104, e.g., in focused irradiation mode 1). The focused irradiation mode 1) may for example be used for pencil beam scanning (e.g., by scanning the ion beam by moving the downstream quadrupole magnet 112B laterally). The actively collimated/fan beam mode 2) can be used for imaging or higher intensity collimated beam delivery, for example using a doublet arrangement. The broad beam illumination 3) may for example be used for imaging and/or passively collimated beam delivery, for which one or more additional collimators may optionally be provided, e.g., as part of the beamline 200 or downstream of the beamline 200 (e.g., between the beamline 200 and the sample holder 201 and/or as part of the sample holder 201. With only very minor modifications of the beamline 200 and/or downstream of the beamline 200, this may for example allow for minibeam irradiation, e.g., by adding a suitable multi-slit collimator, as well as for passively scattered beam delivery or FLASH beam delivery (i.e., irradiation at a highly elevated dose rate on a very short time scale) e.g., by adding a suitable compensator and/or 3D modulator.

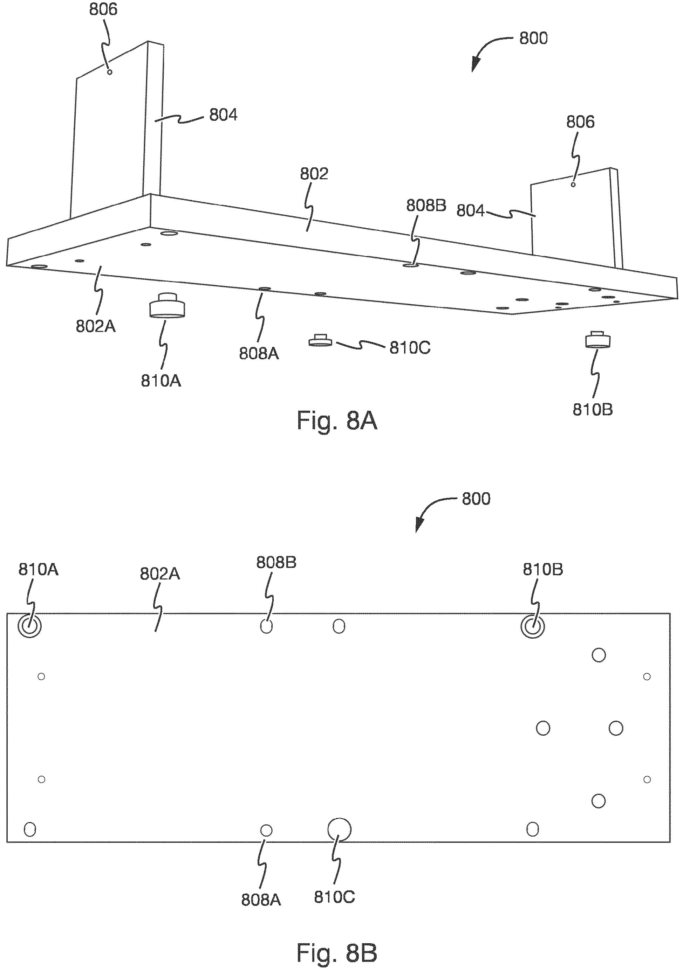

Each of the components of the beamlines 100 and 200 may also be provided independently of the beamlines 100/200 and the other components as a separate unit or component, e.g., for use in a beamline for ion beam irradiation. This applies in particular to the kinematic mount 116 or 300 (and its parts, e.g., the mount portion 116B or 300B as, e.g., shown in Fig. 3B, or the holder portion 116A or 300A together with the intermediate portion 116C or 300C as, e.g., shown in Fig. 3A), the collimator 110 or 400 (and its parts, e.g., the collimator slit of Fig. 4), the energy degrader 108 or 500 of Fig. 5, the supporting platform 600 of Fig. 6 and the alignment template 800 of Figs. 8A/B.

Fig. 3A and 3B depict an adjustable kinematic mount 300 for removably mounting a quadrupole magnet in a transportable beamline according to an example in exploded perspective views. Fig. 3A shows a holder portion 300A and an intermediate portion 300C of the mount 300 while Fig. 3B shows a mount portion 300B of the mount 300. The mount 300 may, e.g., be used for mounting a quadruple magnet in a beamline according to any one of the examples described herein such as for example the beamline 100 of Fig. 1 or the beamline 200 of Fig. 2. A mount such as the mount 300 may for example be used for mounting the central quadrupole magnet 112C of a focusing assembly as in the example of Fig. 3A/B and/or for mounting one or both of the upstream and downstream quadrupole magnets 112A, 112B.

The adjustable kinematic mount 300 is similar to the two-stage kinematic mounts 116 of Fig. 2 and also comprises a holder portion 300A holding (or for holding) the quadrupole magnet 112C (or another quadrupole magnet), a mount portion 300B for coupling to a common base structure (not shown) of the beamline (e.g., via the translation stage(s) 214 and/or 216) and an intermediate portion 300C arranged between the mount portion 300B and the holder portion 300A. The intermediate portion 300C is kinematically and removably coupled to the mount portion 300B at a first plurality or set of three moveable kinematic mount points 118B that allow for adjusting the position and orientation of the intermediate portion 300C (and thereby the holder portion 300A and the quadrupole magnet 112C) with respect to the mount portion 300B (and thereby the common base structure 114 and the collimator (not shown) of the respective beamline) in all six motional degrees of freedom (translation and rotation along/about X, Y and Z). The holder portion 300A is kinematically and removably coupled to the intermediate portion 300C at a second plurality or set of three moveable kinematic mount points 118A that allow for adjusting the position and orientation of the holder portion 300A (and thereby the quadrupole magnet 112C) with respect to the intermediate portion 300C (and thereby the mount portion 300B as well as the common base structure 114 and the collimator of the respective beamline) in all six motional degrees of freedom (translation and rotation along/about X, Y and Z).

The holder portion 300A comprises a holder 302 (e.g., a case or housing) for the quadrupole magnet 112C, which may for example be embodied as a Halbach quadrupole magnet comprising a plurality of magnetic segments arranged around a bore and exhibiting a spatially rotating magnetization pattern for generating a quadrupolar field within the bore.

The holder portion 300A further comprises a magnet base 304 that is attached to the holder 302 (e.g., a bottom surface thereof). The magnet base 304 comprises three contact surfaces 306 (referred to as "upper contact surfaces" below) for the kinematic mount points 118A (e.g., on a bottom surface of the magnet base 304 and thus not visible in Fig. 3A). In the example of Fig. 3A, the three upper contact surfaces 306 are formed by the bottom surface of the magnet base 304 itself. Additionally or alternatively, some or all of the upper contact surfaces 306 may for example also be formed by inserts in the magnet base 304. The contact surfaces 306 may for example be made of brass (e.g., for improved pressure resistance) whereas (the remainder of) the magnet base 304 and/or the holder 302 may for example be made of aluminum (e.g., to facilitate machining and reduce weight). The upper contact surfaces 306 are shaped so as to kinematically constrain the holder portion 300A (e.g., in all six motional degrees of freedom) when placed on the spherical surfaces 312 on the intermediate portion 300C described below. The three upper contact surfaces 306 may for example comprise a concave tetrahedron (or cone), a V-shaped groove and a flat surface (Kelvin-type kinematic mount).

The intermediate portion 300C comprises a tip/tilt plate 308 (e.g., made of brass) that is moveably supported by a base 310 so as to be tiltable and translatable with respect to the base 310 in three directions (i.e., all six motional degrees of freedom). The tip/tilt plate 308 forms or supports three spherical contact surfaces 312 (referred to as "lower contact surfaces" below) for the kinematic mount points 118A that are to engage the upper contact surfaces 306 on the holder portion 300A to kinematically constrain the holder portion 300A. In the example of Fig. 3A, the lower contact surfaces 312 are formed by three spheres, preferably precision-machined spheres arranged on the top surface of the tip/tilt plate 308, which may for example be made of steel. By tilting and/or translating the tip/tilt plate 308, the mount points 118A may be moved (thus making the mount points 118A "moveable mount points").

The intermediate portion 300C further comprises three adjustment screws 314 (preferably micrometer screws) facing the base 310, where the adjustment screws 314 may for example rest on and/or press against corresponding contact surfaces (e.g., made of brass) on the base 310. The adjustment screws 314 may be lockable by a respective locking means such as a clamp or set screw. By adjusting the adjustment screws 314, the tip/tilt plate 308 may be moved (translated) in a first direction (towards or away from the base/in the Y-direction in Fig. 3A) and rotated about a second direction and a third direction parallel to (a top surface of) the tip/tilt plate 308 (e.g., a second direction parallel to a first side of the tip/tilt plate 308, for example the X-direction, and a third direction parallel to a second side of the tip/tilt plate 308, for example Z-direction). The adjustment screws 314 may be arranged so as to be aligned with the lower contact surfaces 312 in the second and third directions and/or so as to form an alternating arrangement with the lower contact surfaces 312 with one of the lower contact surfaces 312 being arranged between two adjustment screws 314 (e.g., along the second direction) and the remaining adjustment screw 314 being arranged between the remaining two contact surfaces 312 (e.g., along the second direction).

The base 310 supports three adjustment screws 320 (e.g., micrometer screws) configured to move (translate) the tip/tilt plate 308 in the second and third directions (X, Z) and tilt (rotate) the tip/tilt plate 308 about the first direction (Y). The adjustment screws 320 may for example rest and/or press against side faces of the tip/tilt plate 308, e.g., pressing the tip/tilt plate 308 against corresponding elastic members (e.g., springs or spring-loaded screws, not shown) on the opposite side of the base 310. The adjustment screws 314 may be lockable by a respective locking means such as a clamp or set screw.

The base 310 comprises three contact surfaces 310A (referred to as "upper contact surfaces" below) for the kinematic mount points 118B (e.g., on a bottom surface of the base 310 and thus not visible in Fig. 3A). The upper contact surfaces 310A are shaped so as to kinematically constrain the intermediate portion 300C (e.g., in all six motional degrees of freedom) when placed on the spherical surfaces 330 on the mount portion 300B described below. The three upper contact surfaces 310A may for example comprise a concave tetrahedron (or cone), a V-shaped groove and a flat surface (Kelvin-type kinematic mount). The base plate 310, in particular its bottom surface and/or the upper contact surfaces 310A, may serve as a reference relative to which the magnetic axis of the quadrupole magnet 112C may be aligned using the tip/tilt plate 308 (for example in step 702 of method 700 described below), e.g., such that the magnetic axis is in a known pre-defined position and orientation relative to this reference.

The mount portion 300B comprises an upper part 326 that forms or supports the first set of mount points 118B and a bottom part 328 for coupling to the common base structure (not shown). The bottom part 328 may for example be attached to a slide of a translation stage for the respective quadrupole magnet, e.g., one of the translation stages 214 to 220 of Fig. 2. The upper part 326 (e.g., the bottom surface thereof, which may be precision-machined) is slidably arranged on the bottom part 328 (e.g., a top surface thereof, which may also be precision-machined), e.g., such that the upper part 326 can slide in the second and third directions (X-Z-plane). Thereby, the kinematic mount points 118B (and thus the intermediate portion 300C and the holder portion 300A with the quadrupole magnet 112C) may be translated in the second and third directions (X, Z) and rotated about the first direction (Y). The upper and bottom parts 326, 328 may be locked to each other by one or more locking means such as screws (i.e., screwed to each other, for example through (e.g., three) oversized holes 336 in the upper part 326) to prevent further sliding of the upper part 326.

The mount portion 300B further comprises a set of three height-adjustable pins 330 having a spherical tip forming three spherical contact surfaces 330A (referred to as "lower contact surfaces" below) for the kinematic mount points 118B that are to engage the three upper contact surfaces 310A for kinematically constraining the intermediate portion 300C. The pins 330 are configured such that their height above the upper part 326 (in the first direction/Y-direction) and thereby the height/position of the kinematic mount points 118B in the Y-direction can be adjusted (thus making the kinematic mount points 118B "moveable"). By adjusting the height of the pins 330, the kinematic mount points 118B (and thus the intermediate portion 300C and the holder portion 300A with the quadrupole magnet 112C) may be translated in the first direction (Y) and rotated about the second and third directions (X, Z). The pins 330 are further configured to be locked with respect to the upper part 326 (e.g., by corresponding locking or fixation means) so as to lock the height/position of the kinematic mount points 118B in the Y-direction, e.g., after adjustment thereof. The spherical tip of the pins 330 may continuously transition into a cylindrical portion 330B of the same diameter, which may for example be used for mechanical alignment with an alignment template such as the alignment template 800 of Fig. 8.

The symmetry axis of the spherical tip/the spherical surface 330A may coincide precisely with the symmetry axis of the remaining parts of the heigh adjustable pins 330, for example to fully decouple adjustment of the mount point height (Y) from the movement in the horizontal plane (X-Z). The spherical tip with the contact surface 330A and/or the cylindrical portion 330B may be a turned part. In one example, the pins 330 may be formed by attaching this part to a dowel pin (preferably a precision dowel pin) prior to turning and then forming the spherical tip with the contact surface 330A and/or the cylindrical portion 330 by turning. This may ensure that the symmetry axes coincide precisely.

Fig. 4 depicts an adjustable collimator aperture 400A/B for a collimator 400 for use in a transportable beamline according to an example in a perspective view. The collimator aperture 400A/B and/or the collimator 400 may, e.g., be used for collimating an ion beam in a beamline according to any one of the examples described herein such as for example the beamline 100 of Fig. 1 or the beamline 200 of Fig. 2.

The collimator aperture 400A/B is formed by a pair of collimator plates 402 defining a slit-like aperture (collimator slit) therebetween, which in the example of Fig. 4 extends along the Y-direction, e.g., for limiting a spatial extent of the ion beam along the X-direction. The collimator plates 402 may for example be made of graphite. Each of the collimator plates 402 is arranged at (e.g,, mounted to or held in) a respective holder 404..

The collimator aperture 400A/B is an adjustable aperture configured to adjust a spacing (aperture/slit width) between the collimator plates 402. For this, one or both of the collimator plates 402 are mounted (e.g., via the holder 404) to a translation stage 406 (e.g., as an example for the actuator/stage 212 in Fig. 2) that is configured to move the collimator plates 402 with respect to each other. Preferably, the translation stage 406 is configured to move the collimator plates 402 by a same distance in opposite directions (e.g. +X and -X) such that the slit width can be adjusted without shifting the center of the aperture (thus maintaining alignment of the collimated ion beam with respect to the magnetic axes of the quadrupole magnets in the focusing assembly, not shown in Fig. 4). In the example of Fig. 4, the translation stage 406 comprises a pair of slides 408 (e.g., carriages) to which the collimator plates 402 are mounted. The slides 408 are arranged on a double-threaded spindle 410 so as to synchronously move by equal, but opposite distances when the spindle 410 is rotated. The spindle 410 is actuated by an actuator 412, e.g., a stepper motor, wherein the actuator 412 is preferably computer-controlled so as to allow for automated operation of the translation stage 406 (e.g., remotely and/or automatically without need for manual intervention within the beamline). The translation stage 406 may be equipped with an encoder.

In some examples, one of the collimator plates 402 (e.g., the right one in the example of Fig. 4) is mounted to another (e.g., linear) translation stage 414 (for example a manually-operated translation stage) arranged on the respective slide 408. The translation stage 414 is configured to move that collimator plate 402 with respect to the other collimator plate 402 to adjust a center of the collimator aperture 400A/B (e.g., to align the center of the collimator aperture 400A/B to the magnetic axis of the quadrupole magnets in the focusing assembly). Preferably, the translation stage 414 is configured to move the collimator plate 402 along the same direction as the translation stage 406 (e.g., parallel to the spindle 410). The translation stage 414 may be lockable, e.g., to fix the center of the collimator aperture 400A/B.

The collimator aperture 400A/B may comprise a second pair of collimator plates (not shown) defining a second slit therebetween. The second pair of collimator plates may be arranged adjacent to the first pair of collimator plates 402 along the beam path but oriented at an angle (preferably perpendicular) to the first pair of collimator plates 402, e.g., such that the two collimator slits are rotated by 90° with respect to each other. This may be achieved by providing a second assembly as the one shown in Fig. 4 and rotating this second assembly by 90 degrees about the beam axis with respect to the (first) assembly shown in Fig. 4. The two pairs of collimator plates may thus collectively define a rectangular collimator aperture. Preferably, the second pair of collimator plates is also adjustable so as to allow for adjusting the width of the second slit (thus allowing for adjusting both the width (X) and the height (Y) of the collimator aperture 400A/B). The second pair of collimator plates may for example also be arranged on a translation stage similar to the translation stage 406 (but oriented at an angle and in particular perpendicular thereto).

The collimator 400 may comprise a pair of collimator apertures, e.g., a second (or downstream) collimator aperture 400B that is displaced with respect to a first (or upstream) collimator aperture 400A along the beam path (e.g., similar to the pair of apertures 110A, 110B in Fig. 2), for example to define the spatial extent and the divergence of the ion beam. Each of the first and second collimator apertures 400A, 400B may be embodied similar to the collimator aperture 400A/B of Fig. 4 as described above and may also comprise two pairs of adjustable collimator plates configured to move by a same distance in opposite directions for adjusting a size of the second collimator aperture.

Fig. 5 depicts an energy degrader 500 for use in a transportable beamline according to an example in a perspective view. The energy degrader 500 may, e.g., be used for decelerating ions in an ion beam in a beamline according to any one of the examples described herein such as for example the beamline 100 of Fig. 1 or the beamline 200 of Fig. 2.

The energy degrader 500 comprises a pair of wedge-shaped degrading members 502A, 502B, which may for example be made of boron carbide (B₄C). The degrading member 502A, 502B are configured to be arranged in the beam path for the ions to pass therethrough such that the ions are decelerated by interaction with the material of the degrading members 502A; 502B.

The energy degrader 500 is an adjustable energy degrader that is configured to decelerate ions from a given initial energy (e.g., between 50 MeV/mᵤ and 100 MeV/mᵤ, for example 70 MeV/mᵤ) to an adjustable target energy (e.g., across the range of 20 MeV/mᵤ to 50 MeV/mᵤ). For this, the degrading members 502A, 502B are moveable with respect to each other in a lateral direction perpendicular to the beam path (e.g., the X-direction in the example of Fig. 5) to adjust a thickness/amount of material that the ion beam passes through (i.e., a length of the beam path within/through the degrading members 502A, 502B). One or both of the degrading members 502A, 502B may for example be mounted to a translation stage 506 (e.g., as an example for the translation stage 210 of Fig. 2) configured to adjust a lateral overlap of the degrading members 502A, 502B (e.g., along the X-direction) by moving the degrading members 502A, 502B with respect to each other.

The degrading members 502A, 502B may in particular be mounted similar to the collimator plates 402 of the adjustable collimator aperture 400A/B of Fig. 4, for example in respective holders 504) mounted to slides 508 on a double threaded spindle 510 of the translation stage 506, which is actuated via (e.g., computer-controlled) actuator 512. One of the degrading members 502A, 502B, e.g., the left degrading member 502A in the example of Fig. 5, may be mounted to another translation stage 514 on the respective slide 508, e.g., for adjusting a center of the energy degrader 502 (e.g., with respect to the magnet axes of the quadrupole magnets in the focusing assembly).

In some examples, the energy degrader 500 further comprises a scattering foil 516 (e.g., made of tantalum, for example if high scattering is desired, or of a material with a mixture of elements of both low and high nuclear charge (Z) if a differential degree of scattering is desired) that is arrangeable in the beam path for scattering the ion beam. For moving the scattering foil 516 into and out of the beam path, the scattering foil 516 may be mounted on a translation stage. In the example of Fig. 5, the scattering foil is mounted to the translation stage 506 (e.g., via holder 518), in particular to the same slide 508 to which one of the degrading members 502A, 502B (e.g., the degrading member 502B that is not mounted to the additional stage 514) is mounted. The scattering foil 516 may be arranged such (e.g., laterally displaced with respect to the degrading member 502B) that the energy degrader 500 can assume a first ("closed") configuration (e.g., for a focused irradiation mode) in which the degrading members 502A, 502B are arranged in the beam path and the scattering foil 516 is not and a second ("open") configuration (e.g., for broad beam irradiation) in which the scattering foil 516 is arranged in the beam path and the degrading members 502A, 502B are not (e.g., by moving apart the degrading members 502A, 502B such that they no longer overlap with each other and moving the scattering foil 516 into the beam path). In other examples, the scattering foil 516 may not be part of the energy degrader but may be provided separately therefrom and arranged elsewhere within the beamline.

Fig. 6 depicts a supporting platform 600 for a transportable beamline according to an example in an exploded perspective view. The supporting platform 600 may, e.g., be used for supporting (e.g., mounting thereto) a beamline (or at least a part thereof) according to any one of the examples described herein such as for example the beamline 100 of Fig. 1 or the beamline 200 of Fig. 2. The supporting platform 600 may be provided separate from the beamline or as part of the beamline.

The supporting platform 600 comprises an upper part 602 for mounting the beamline thereon and a lower part (or base) 604 supporting the upper part 602. In the example of Fig. 6, the upper part 602 is an optical table having an internal (e.g., metal) honeycomb structure. The optical table 602 provides a flat, stable surface for the beamline (at comparably low weight).

The base 604 comprises a frame 604A/B supporting the upper part/optical table 602, which in the example of Fig. 6 is embodied as a two-part frame comprising an upper frame part 604A supporting the optical table 602 and a lower frame part 604B supporting the upper frame part 604A. The base 604 (e.g., the frame 604A/B, in particular the upper frame part 604A) may be equipped with casters 606 (which may be removable), for example to move (e.g., for aligning) the supporting platform 600 with the beamline relative to an external ion source (e.g., in the second and third directions/the X-Z plane). The lower frame part 604B (which may be removable from the upper frame part 604A and, in the example of Fig. 6, is embodied as a pair of traverses), comprises height-adjustable feet 612. The height-adjustable feet 612 may be extended and retracted in the first direction/Y-direction so as to move the optical table 602 along the first direction (Y) and rotate the optical table 602 about the second and third directions (X-Z). The height-adjustable feet 612 may be configured to be extended so as to lift off the casters 606 from the ground (such that the frame 604A/B can no longer be moved horizontally/in X-Z).

The upper part/optical table 602 is moveably (e.g., slidably) arranged on the base 604 (e.g., on the upper frame part 604A), in particular such that the optical table 602 can be moved horizontally (in the second and/or third directions, X and/or Z) with respect to the base 604 (thus, e.g., allowing for translation in X and Z and rotation about Y). In this way, the supporting platform 600 may be configured to adjust all six motional degrees of freedom (X, Y, Z) of the optical table 602. In the example of Fig. 6, the optical table 602 is supported by the base 604 (e.g., the upper frame part 604A) via a plurality of slide plate pairs 608.. The slide plate pairs 608 may each comprise one or more adjustment screws configured to slide (e.g., shift or displace) the optical table 602 with respect to the base 604 along one or both of the second and third directions (X and/or Z).

Fig. 7 shows a flow diagram of a method 700 of aligning a beamline for ion beam irradiation according to an example. The method 700 may for example be used to align a transportable beamline for ion beam irradiation according to any one of the examples described herein such as for example the beamline 100 of Fig. 1 or the beamline 200 of Fig.. The method 700 is not limited to the order of execution implied by the flow diagram of Fig. 7. As far as technically feasible, the method 700 may be executed in an arbitrary order and steps thereof may also be executed simultaneously at least in part.

The method 700 comprises mounting the collimator and the focusing assembly of the beamline to the common base structure, e.g., by executing step 704. The method 700 further comprises, for each of the quadrupole magnets of the focusing assembly, aligning a magnetic axis of the quadrupole magnet to the beam path for the ion beam by adjusting a position and/or an orientation of the quadrupole magnet relative to the common base structure and fixing the position and the orientation of the quadruple magnet relative to the common base structure, e.g., by executing some or all of steps 702, 706, 706A and 706B. Optionally, the method 700 may further comprise aligning the collimator and/or the degrader of the beamline relative to the magnetic axes of the quadrupole magnets, e.g., by executing step 708, and/or mounting the beamline to a supporting platform and/or aligning the beam path of the beamline to an ion beam of an ion beam source, e.g., by executing step 710.

The method 700 may for example be used for aligning the beamline prior to first use, e.g., when assembling or setting up the beamline for the first time. Additionally or alternatively, the method 700 may also be used for re-aligning the beamline at a later point, for examples during use as required (e.g., when determining that re-alignment is needed) or after transport (e.g., when assembling or setting up the beamline at a new ion beam source facility for the first time or when reinstalling the beamline at the ion source after temporarily storing away the beamline) or at regular intervals such as prior to each use, although by design of the beamline according to the invention, the latter use cases typically will not be required.

In the following, the method 700 is described using the beamline 200 of Fig. 2 with the kinematic mounts 300 of Fig. 3, the collimator 400 of Fig. 4, the energy degrader 500 of Fig. 5 and the supporting platform 600 of Fig. 6 as non-limiting examples for illustration purposes.

In optional step 702, the magnetic axes of each of the quadrupole magnets 112A-C are aligned relative to the intermediate portion 300C of the respective kinematic mount 300, e.g., such that the magnetic axis of the quadrupole magnet is at a pre-defined position and orientation (nominal position and orientation) relative to the intermediate portion 300C. For example, the base 310 (in particular its bottom surface and/or the upper contact surfaces 310A for the kinematic mount points 118B) may be used as a reference relative to which the magnetic axis is aligned (e.g., such that the magnetic axis is in a known and well-defined position and orientation relative to the first set of mount points 118B). The magnetic axis may for example be aligned to be within 50 µm, preferably 20 µm, most preferably 10 µm of the pre-defined position and orientation (which may be much closer than what can typically be achieved during fabrication of the magnet, e.g., due to inhomogeneities in the magnetic material). Preferably, the alignment of the magnetic axes relative to the intermediate portion 300C is performed prior to mounting the quadrupole magnets to the common base structure in step 704.

The magnetic axis may be aligned relative to the intermediate portion 300C by adjusting one or more mounts points of the second set of mount points 118A, for example by translating and/or rotating the tip/tilt plate 308 about one or more of the first, second and third directions (X, Y, Z) using the adjustment screws 314 and 320. Once the magnetic axis is aligned, the position and orientation of the quadrupole magnet relative to the intermediate portion 300C is fixed, e.g., by locking the adjustment screws 314, 320.. Step 702 may comprise determining the magnetic axis, the magnetic moments and/or pole orientation of the quadrupole magnets, for example using a stretched-wire machine (e.g., by positioning a stretched wire at the magnetic axis and/or moving the stretched wire relative to the magnetic axis). For aligning the magnetic axis relative to the intermediate portion 300C, the intermediate portion 300C may be placed (e.g., in the stretched-wire machine) on a (fixed) reference plate with precision-machined contact surfaces corresponding to the arrangement of spherical surfaces 330A of the pins 330. The position of the reference plate (e.g., relative to the stretched wire) may be measured and a deviation of the position and orientation of the magnetic axis relative to its nominal position and orientation determined therefrom (and corrected for by adjusting the mount points 118A.

In step 704, the collimator 110/400 and the focusing assembly 112 with the quadrupole magnets 112A-C is mounted to the common base structure 114. This may for example comprise attaching the translation stages 406 (e.g., a pair of orthogonal translation stages for each of the upstream and downstream apertures 110A/400A, 110B/400B) and the translation stages 214 to 220 to the common base structure 114 and mounting the pair of apertures 110A/400A, 110B/400B (e.g., the holders 404 thereof) and the kinematic mounts 300 with the quadrupole magnets 112A-C (e.g., the bottom parts 328 of the mount portions 300B) to the respective translation stage (e.g., the slide thereof). The collimator 110/400 and the focusing assembly 112 may be mounted to the common base structure 114 at the same time or at different points in time, e.g., the focusing assembly 112 between steps 702 and 706 and the collimator 110/400 between steps 706 and 708. Step 704 may comprise assembling the kinematic mounts 300, for example by placing the intermediate portion 300C on the mount portion 300B (at the first set of kinematic mount points 118B, thereby kinematically constraining the intermediate portion 300C) and placing the holder portion 300B with the respective quadrupole magnet 112A-C on the intermediate portion 300C (at the second set of kinematic mount points 118A, thereby kinematically constraining the holder portion 300A). Step 704 may further comprise mounting the common base structure 114 on the base plate 226 in a housing 200 (e.g., on the guiding structures 228) and/or on the supporting platform 600.

In step 706, the magnetic axis of each of the quadrupole magnets 112A-C is aligned to the beam path, e.g., such that the magnetic axes exactly coincide (i.e., being colinear) with each other and with the beam path (e.g., as defined by entrance and exit apertures of the beamline 200 or by the collimator 110/400). It is to be noted that the collimator 110/400 at this point may not be mounted or aligned yet but rather the magnetic axes of the quadrupole magnets themselves may define the (future) beam path to which the collimator 110/400 is then aligned at a later point, e.g., as detailed below). As such, the alignment of the magnetic axes of the quadrupole magnets 112A-C to each other in itself may constitute an "alignment of the magnetic axes to the beam path" as used herein.

The magnetic axes of the quadrupole magnets 112A-C are aligned to the beam path by adjusting one or more kinematic mount points of the adjustable kinematic mounts 300, in particular one or more (e.g., all) of the first set of kinematic mount points 118B (coupling the mount portion 300B and the intermediate portion 300C). As detailed above with reference to Fig. 3B, the kinematic mount points 118B may be moved by adjusting the height of the height-adjustable pins (translation in first direction/Y, rotation about second and third directions/X and Z) and/or shifting the upper part 326 of the mount portion 300B with respect to the bottom part 328 (translation in second and third directions/X and Z, rotation about first direction/Y).

The mount points 118B may for example be adjusted using a mechanical alignment template (e.g., a jig), which may be provided as part of (i.e., comprise in) the beamline or separate from the beamline. The alignment template may comprise a plurality of precision holes (or recesses) that are arranged in a pattern corresponding to the nominal positions (e.g., in the second and third directions/in the X-Z plane) of some or all of the mount points 118B (e.g., two mount points 118B and/or as many as required for constraining all motional degrees of freedom in X-Z or Y-X-Z) for each of the kinematic mounts 300. The precision holes may be configured to receive a respective one of the mount points 118B (i.e., the corresponding pin 330, in particular the spherical tip 330A and/or the abutting cylindrical portion 330B) and may be adapted to precisely match a physical dimension (e.g., a diameter) thereof (e.g., for a location clearance fit). The precision holes may thus be used for adjusting the mount points 118B in the second and third directions (translation in second and third directions/X and Z, rotation about first direction/Y). The alignment template may further comprise a flat precision surface (e.g., on its bottom) which may be used for adjusting the mount points 118B in the first/Y direction (translation in first direction/Y, rotation about second and third directions/X and Z).

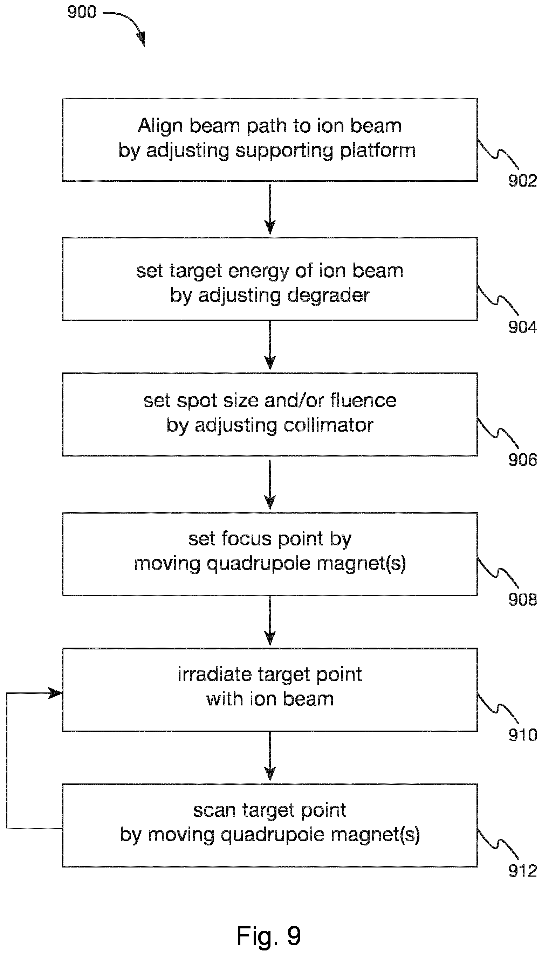

An example for such a mechanical alignment template 800 is shown in Figs. 9A and 9B, with the alignment template 800 being depicted in a perspective view in Fig. 8A and in bottom view in Fig. 8B. The alignment template 800 comprises a base plate 802 (e.g., made of aluminum) with a (preferably precision machined) flat bottom surface 802A. The alignment template 800 further comprises a pair of (optionally removable) side pieces 804 at each end of the base plate 802 (e.g., along the beam path/longitudinal direction as defined by the hole pattern), wherein each of the side pieces comprises a reference marker 806 for absolute alignment of the template 800 within the beamline 200. In the example of Fig. 8A, the reference markers 806 are optical reference markers for alignment with an alignment laser beam (line laser), namely a pair of holes/apertures (having a diameter on the order of the desired absolute positioning accuracy within the beamline, for example a diameter between 0.5 mm and 2.0 mm, e.g., 1.0 mm).

In the flat bottom surface 802A, the alignment template 800 comprises a plurality of precision holes or recesses 808A, 808B, each of which is arranged at a relative nominal position of a respective one of the kinematic mount points 118B/pins 330 and configured to register the spherical tip 330A and the cylindrical portion 330B thereof (being precisely adapted to a physical dimension thereof). The precision holes or recesses 808A, 808B may comprise matching holes or recesses 808A precisely matching the physical dimensions of the spherical tip 330A and the cylindrical portion 330B in both directions (e.g., circular holes) as well as partially over-sized holes 808B precisely matching the physical dimension of the spherical tip 330A and the cylindrical portion 330B in only one direction (e.g., elongated or long holes). The template 800 may for example comprise at least one matching hole 808A and at least one partially oversized hole 808B for each kinematic mount 300/ quadrupole magnet 112A-C.

The alignment template 800 further comprises a plurality of inserts 810A-C (which may, e.g., be made of brass), for example one for each kinematic mount 300 (i.e., three in the example of the beamline 200). Each of the inserts is configured to be inserted into one of the precision holes 808A and/or 808B. Each of the inserts forms an (upper) contact surface for the kinematic mount points 118B, which may for example mimic a respective one of the contact surfaces 310A on the bottom of the base 310 of the intermediate portion 300C. The template 800 may comprise two inserts 810A, 810B with a concave tetrahedron (or cone) as the contact surface (e.g., to fully constrain the respective pin parallel to the flat bottom surface 802A/the X-Z plane) and one insert 810C with a flat contact surface (e.g., to only constrain the respective pin perpendicular to the flat bottom surface 802A/the Y direction while allowing for motion in the X-Z plane). The inserts 810A-C may be formed such that all of these contact surfaces are positioned at precisely the same distance from the flat bottom surface 802A when inserted into one of the precision holes 808A, 808B.

The alignment of the magnetic axes in step 706 may be performed in two steps (which, optionally, may be iterated, e.g., until a desired alignment accuracy is achieved). In a first step 706A, a single mount point 118B/pin 330 (referred to as the first mount point/pin) may be adjusted for each of the quadrupole magnets, e.g., for absolute positional alignment of the quadrupole magnets 112A-C with respect to each other and within the beamline 200. In a second step 706B, all of the mount points 118B/pins 330 may then be adjusted for each of the quadrupole magnets 112A-C, e.g., for relative alignment of the orientation and/or position of the quadrupole magnets 112A-C with respect to each other and within the beamline 200. This may for example be done as follows:

In step 706A, the first mount point/pin of each of the kinematic mounts 300 may be adjusted to a nominal height above the common base structure 114 along the first/Y direction and the first mount point/pin of at least two of the kinematic mounts 300 (corresponding to a first and second magnet as described below) may be adjusted to its nominal position along the second and third directions (X-Z).

For this, the inserts 810A-C (one for each mount 300) may be inserted into the alignment template 800 with the inserts 810A and 810B (with the concave tetrahedron or conical contact surface) being placed in a hole 808A, 808B corresponding to a first quadrupole magnet (preferably the upstream quadrupole magnet 112A) and a second quadrupole magnet (preferably the downstream quadrupole magnet 112B), e.g., as illustrated in Fig. 8B. For the first and second quadrupole magnets 112A/B, the upper part 326 and the bottom part 328 of the mount portion 300B of the respective mount 300 may be unlocked (e.g., by loosening locking screws in the oversized holes 336) such that the upper part 326 can slide on the bottom part 328. When placing the alignment template 800 on the mount portion 300B, the respective first pin 330 may thus slide into position in the insert 810A/810B (being kinematically constrained therein for alignment along all three directions) with the first pin 330 of the remaining magnet, e.g., resting on the flat contact surface of the insert 810C (for height adjustment only).

For each of the quadrupole magnets 112A-C, the height of the first mount point/pin may be adjusted such that the alignment template 800 (e.g., the base plate 802 or the flat bottom surface 802A thereof) is oriented horizontally (perpendicular to Y). For this, the reference holes 806 may for example be aligned to the height of a horizontal line laser (e.g., provided as part of the ion beam source), which may previously have been aligned to entrance and exit apertures of the beamline 200 (e.g., by eye). Thereby, the first mount points/pins for the quadrupole magnets 112A-C may be adjusted to their nominal height along the first direction (Y).

By sliding the upper parts 326 of the mount portions 300B of the mounts 300 for the first and second quadrupole magnets 112A/B on the respective bottom part 328, the holes 806 may be aligned horizontally to the line laser (e.g., such that the line laser passes through both holes 806 to horizontally and vertically align the holes 806 and thereby the alignment template 800). Thereby, the first mount points/pin for the first and second quadrupole magnets 112A/B may be adjusted to their nominal positions along the second and third directions (X-Z). The upper part 326 and the bottom part 328 of the mount portion 300B of the mounts 300 for the first and second quadrupole magnets 112A/B may then be locked again (e.g., by tightening the locking screws in the oversized holes 336).

In step 706B, the remaining mount points/pin of the first set of mount points 118 are adjusted to the nominal height (Y) above the common base structure 114 and to their nominal positions in the second and third directions (X-Z) for each of the kinematic mounts 300.

For this, the alignment template 800 may be placed on the mount portions 300B of the kinematic mounts 300 for the quadrupole magnets 112A-C without the inserts 810A-C, for example such that the flat bottom surface 802A is in contact with first mount points/pin (whose height was already adjusted in step 706A). The height of the remaining mount points 118B/pins 330 may then be adjusted such that all of the pins 330 touch the flat bottom surface 802A. In this configuration, the magnetic axes of all quadrupole magnets 112A-C would lie parallel to the horizontal plane (X-Z) and at exactly the same height along the first/Y direction (e.g., aligned with the centers of the entrance and exit apertures of the beamline 200).

The upper part 326 and the bottom part 328 of the mount portion 300B of the mounts 300 for all quadrupole magnets 112A-C may be unlocked (e.g., by loosening locking screws in the oversized holes 336) such that the upper part 326 can slide on the bottom part 328. The alignment template 800 may then be placed on the mount portions 300B in such a way that the pins 330 (e.g., the spherical tip 330A and/or the abutting cylindrical portion 330B of the same diameter) are arranged in (e.g., slide into) the corresponding precision hole/recess 808A, 808B and the alignment template 800 is at its nominal position within the beamline (e.g., the reference holes 806 are aligned with the line laser), thereby adjusting the mount points 118B/pin 330 to their nominal horizontal positions (X-Z). The holes/recesses 808A, 808B are arranged such that they uniquely define the positions and orientations of the first set of mount points 118B. The upper part 326 and the bottom part 328 of the mount portion 300B of the mounts 300 may then be locked again (preferably without lifting off the alignment template 800) to fix the positions and orientations of the first set of mount points 118B. Relative alignment between the first set of mount points 118B may now be as accurate as the machining accuracy of the alignment template 800.

In optional step 708, the collimator 110/400 and, optionally, the energy degrader 500 may then be aligned to the (coinciding) magnetic axes of the quadrupole magnets 112A-C, e.g., such that the centers of the apertures 110A/400A, 110B/400B are colinear with the magnetic axes and the beam path (as defined by the magnetic axes) passes through the degrading members 502A, 502B.

The alignment may for example be made using an optical telescope (e.g., a precision metrology telescope with, optionally, crosshairs). The optical axis of the telescope may be aligned to the magnetic axes of the quadrupole magnets 112A-C (e.g., so as coincide/be colinear therewith). For this, the alignment template 800 (with or preferably without the inserts 810A-C) may again be placed on the pins 330 of the (locked) mount portions 300B of the mounts 300 (e.g., such that the pins 330 are arranged in their respective hole/recess 808A, 808B or the contact surfaces of the inserts 810A-C rest on first pins). The alignment template 800 may be configured such that reference holes 806 in this configuration (i.e., on the correctly aligned pins 330 of the mount portions 300B) are exactly aligned with the magnetic axes of the quadrupole magnets 112A-C in the holder portions 300A (if placed on the mount portions 300B), e.g., with a deviation of at most 50 µm, preferably at most 20 µm, most preferably at most 10 µm. The optical axis of the telescope may then be aligned to the reference holes 806 on the alignment template 800.

The collimator apertures 400A, 400B may then be aligned to the optical axis of the telescope. For this, the center of the apertures 400A, 400B may be adjusted using the respective translation stage 414 (moving one of the collimator plates 402 but not the other collimator plate), e.g., such that the collimator plates 402 are symmetric about the optical axis of the telescope (e.g., the crosshairs). This procedure may be repeated multiple times for successively smaller aperture sizes/slit width (adjustable with the respective translation stage 406), e.g., starting at an aperture size of the order of 1 mm and reducing this until the desired alignment accuracy is achieved.

The energy degrader 500 can for example be aligned in the same way. The alignment of the energy degrader 500 is, however, not particularly critical and it may be sufficient to simply place the energy degrader 500 such that the degrading members 502A, 502B are arranged in and approximately centered to the beam path. The spacing between the degrading members 502A, 502B may be calibrated as a function of the position of the stage 506. The optical telescope may also be used to align other components to the magnetic axes of the quadrupole magnets 112A-C, for example the sample holder 201 and/or additional collimator(s).

After step 708, the internal alignment of the beamline 200 may be complete and the beamline 200 may be ready for use. In optional step 710, the beamline 200 may be mounted to the adjustable supporting platform 600 (e.g., in case this was not already done in step 704 or the beamline 200 was removed from the supporting platform 600 (e.g., for transport). A position and/or an orientation of the supporting platform 600 may then be adjusted to align the beam path 104 of the beamline 200 (e.g., the entrance and exit apertures thereof) to an ion beam of an ion beam source that the beamline 200 is to be used with. The alignment relative to the ion beam source is again not particularly critical since the collimator 110/400 provides an internal reference ("internal beam source") whose alignment relative to the quadrupole magnets 112A-C is fixed and maintained by the common base structure 114 (and the kinematic mounts 300 in case of removal of the magnets). The beamline 200 can thus be aligned relative to the ion beam source by eye (e.g., using line lasers of the ion beam source and reference markers (e.g., marker lines or spots) on the beamline 200 (e.g., the housing thereof). The position and/or orientation of the supporting platform 600 may for example be adjusted using the casters 606 (e.g., for a first coarse alignment in X-Z), the height-adjustable feet 612 (for alignment along Y) and/or the slide plate pairs 608 (for fine alignment in X-Z).

Fig. 9 shows a flow diagram of a method 900 of irradiating a target point using a transportable beamline according to an example. The method 900 may for example be executed using a transportable beamline for ion beam irradiation according to any one of the examples described herein such as for example the beamline 100 of Fig. 1 or the beamline 200 of Fig. 2 with the beamline 200 being used as a non-limiting example for illustration purposes in the following. The method 900 is not limited to the order of execution implied by the flow diagram of Fig. 9. As far as technically feasible, the method 900 may be executed in an arbitrary order and steps thereof may also be executed simultaneously at least in part. The method 900 may be modified in various ways, e.g., by omitting some of steps 902 to 912 (for example some or all of steps 902, 904, 906 and 912).

The method 900 may be executed as part of the method 700 (e.g., with step 902 being executed as part (or instead) of step 710 and steps 904 to 910 being executed subsequently). Additionally or alternatively, the method 900 may also be executed separately and independently of method 700, e.g., during use of the respective beamline (while method 700 may for example only be executed once prior to first use or irregularly).

In optional step 902, the beam path 104 of the beamline 200 is aligned to an ion beam 102 of an ion beam source, e.g., at a clinical facility for particle therapy. This may for example be done using an adjustable supporting platform such as the supporting platform 600 of Fig. 6, e.g., as in step 710 of the method 700. As mentioned above, this alignment is not particularly critical and in particular does not require any internal alignment of the beamline 200 (which may instead have been aligned internally beforehand, e.g., using the method 700) such that the alignment in step 902 is a straightforward procedure that can be accomplished rather quickly. In some examples, the beam path 104 may already have been aligned to the ion beam 102 beforehand.

In step 904, a target energy of the ion beam 102 may be set by adjusting the energy degrader 108, for example by adjusting an overlap between the wedge-shaped degrading members 108A, 108B to change a thickness/amount of material traversed by the ion beam. The target energy may for example be adjusted within a range of 20 MeV/mᵤ to 50 MeV/mᵤ.

In step 906, a spot size (e.g., a diameter or width) and/or a fluence/intensity (e.g., a number of ions per unit of time or per unit of time and area) of the ion beam 102 (e.g., at the target point 106) may be set by adjusting the collimator 110, for example by adjusting an aperture size of one or both of the apertures 110A, 110B (e.g., by moving some or all of the pairs of collimator plates 402 close to or further away from each other using the translation stages 406 to adjust a slit width/height of the collimator apertures).

In step 908, the focus point F of the ion beam 102 is set using the focusing assembly. The focus point F may in particular be adjusted to the target energy set in step 904, e.g., to move or maintain the focus point F to/in a same transverse plane perpendicular to the beam path 104/at the same position along the beam path 104 after changing the target energy in step 904.

The focus point F may in particular be set by moving one or more of the quadrupole magnets 112A-C in the focusing assembly 112. The focus point F may for example be set by adjusting the upstream drift length Do and/or the downstream drift length D2 (while, optionally, maintaining the central drift length D₁). For example, the upstream and central quadrupole magnets 112A, 112C may simultaneously be moved longitudinally along the beam path 104 relative to the collimator 110 and the downstream quadrupole magnet 112B (e.g., using the first translation stage 214 to which both the upstream and central quadrupole magnets 112A, 112C are mounted) to adjust the upstream drift length Do between the collimator 110 and the upstream quadrupole magnet 112A and the downstream drift length D2 between the central quadruple magnet 112C and the downstream quadrupole magnet 112B while maintaining the central drift length D1 between the upstream and central quadrupole magnets 112A, 112C.

In some examples, setting the focus point F of the ion beam 104 may comprise: coarse-adjusting the focus point F by simultaneously moving the upstream and central quadrupole magnets 112A, 112C longitudinally along the beam path 104 relative to the collimator 110 and the downstream quadrupole magnet 112B to adjust the upstream drift length Do and the downstream drift length D2 while maintaining the central drift length D1; and fine-tuning the focus point F by moving the central quadrupole magnet 112C longitudinally along the beam path 104 relative to the upstream quadrupole magnet 112A to (e.g., simultaneously) adjust the central drift length D1 and the downstream drift length D2 (e.g., using the third translation stage 216).

In step 910, the target point 106 may be irradiated with the ion beam 104, e.g., by generating a corresponding control signal for the ion beam source. This may comprise monitoring and/or controlling a delivered dose (e.g., using a monitoring chamber such as an ionization chamber). In one example, the target point 106 is irradiated at different target energies, spot sizes and/or fluences (e.g., for intensity-modulated pencil beam delivery), for example by repeating steps 904, 906 and/or 908 to re-set the target energy, spot size and fluence while maintaining the focus point F.

In step 912, the target point 106 may be moved laterally in a plane perpendicular to the beam path 104, e.g., to a new target point 106 for subsequent irradiation by the ion beam (by the repeating step 910). The target point 106 may be moved by laterally moving one or more of the quadrupole magnets 112A-C, preferably (e.g., only) the downstream quadrupole magnet 112B, with respect to the beam path 104 (e.g., using the translation stage 218/220). By laterally moving the quadrupole magnet(s), the ion beam 104 may be deflected (e.g., as a result of an effective dipole component being introduced due to the resulting misalignment, thus imparting a lateral kick to the ions).

Steps 910 and 912 may be executed repeatedly, e.g., to sequentially scan the target point 106 in the plane perpendicular to the beam path 104. The target point 106 may be scanned along a one-dimensional or two-dimensional scan path (e.g., for line or raster scanning), for example across a two-dimensional target area (which may have an arbitrary shape and, e.g., need not even be simply connected).

In step 912, additionally or alternatively to moving/scanning the target point relative to the sample/target (e.g., a mouse), the sample/target can also be moved or scanned (relative to the target point), for example by moving the target holder 201 in a plane perpendicular to the beam direction, e.g., using the (e.g., motorized) stages 201A, 201B in X and Y direction.

The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

100 - transportable beamline
102 - ion beam
104 - beam path
106 - target point
108 - energy degrader
108A, 108B - degrading member
110 - collimator
110A - upstream collimator aperture
110B - downstream collimator aperture
112 - focusing assembly
112A - upstream quadrupole magnet
112B - downstream quadrupole magnet
112C - central quadrupole magnet
114 - common base structure
116 - kinematic mount
116A - holder portion
116B - mount portion
116C - intermediate portion
118 - kinematic mount points
118A - second set of kinematic mount points
118B - first set of kinematic mount points
120 - supporting platform
200 - transportable beamline
201 - sample holder
210A/B - sample holder translation stages
202 - housing
204 - entrance window
206 - outlet of ion beam source
208 - exit window
210 to 220 - translation stages
222 - radiation shielding plate
224 - beam shaping aperture
226 - base plate
228 - guiding structure
230 - stop
300 - adjustable kinematic mount
300A - holder portion
300B - mount portion
300C - intermediate portion
302 - holder
304 - magnet base
306 - upper contact surfaces for first set of kinematic mount points 118B
308 - tip/tilt plate
310 - base
312 - spherical contact surfaces
314, 320 - adjustment screw326 - upper part of mount portion 300B
328 - bottom part of mount portion 300B
330 - height-adjustable pin
330A - spherical tip/spherical surface
330B - cylindrical portion336 - oversized hole
400 - collimator
400A/B - collimator aperture
402 - collimator plate
404 - holder for collimator plate 402
406 - translation stage
408 - slide
410 - double-threaded spindle
412 - actuator
414 - translation stage
500 - energy degrader
502A, 502B - degrading member
504 - holder for degrading member 502A, 502B
504A - clamp for degrading member 502A, 502B
506 - translation stage
508 - slide
510 - double-threaded spindle
512 - actuator
514 - translation stage
516 - scattering foil
518 - holder for scattering foil 516
600 - adjustable supporting platform
602 - upper part of supporting platform 600
604 - lower part of supporting platform 600
604A - upper frame part
604B - lower frame part
606 - caster
608 -slide plate pair
612 - height-adjustable feet
800 - mechanical alignment template
802 - base plate
802A - flat bottom surface
804 - side piece
806 - reference maker/hole
808A - matching hole
808B - partially oversized hole
810A, 810B - insert with concave tetrahedral contact surface
810C - insert with flat contact surface
D1 - upstream drift length
D2 - central drift length
D1 - downstream drift length
DF - working distance
F - focus point
L - length

## Claims

1. A transportable beamline (100, 200) for ion beam irradiation, in particular small animal ion beam irradiation, the beamline (100, 200) being configured to receive an ion beam (102), in particular a proton beam, from an ion beam source and to guide the ion beam (102) along a beam path (104) to a target point (106) that is to be irradiated, wherein the beamline (100, 200) comprises:
an energy degrader (108, 500) for decelerating ions in the ion beam (102);
a collimator (110, 400) for collimating the ion beam (102);
a focusing assembly (112) for focusing the ion beam (102), the focusing assembly (112) being arranged downstream of the energy degrader (108, 500) and the collimator (110, 400) along the beam path (104) for the ion beam (102) and comprising two or more quadrupole magnets (112A-C) arranged along the beam path (104); and
a common base structure (114) to which at least the collimator (110, 400) and the focusing assembly (112) are mounted, the common base structure (114) being configured to preserve relative alignment between the collimator (110, 400) and the two or more quadrupole magnets (112A-C) during transport of the beamline (100, 200), wherein some or all of said two or more quadrupole magnets (112A-C) are removably mounted to the common base structure (114) via a respective kinematic mount (116, 300).

2. The transportable beamline (100, 200) of claim 1, wherein said common base structure (114) is formed of a single piece of material, in particular a single piece of material having a coefficient of linear thermal expansion of no more than 15 · 10⁻⁶/K and preferably no more than 10 · 10⁻⁶/K, most preferably a single piece of granite, a carbon fiber compound and/or an iron-nickel alloy.

3. The transportable beamline (100, 200) of claim 1 or 2, wherein some or all of said kinematic mounts (116, 300) are adjustable kinematic mounts that are configured to adjust a position and/or orientation of the respective quadrupole magnet (112A-C) relative to the common base structure (114).

4. The transportable beamline (100, 200) of claim 3, wherein some or all of said adjustable kinematic mounts (116, 300) comprise a mount portion (116B, 300B) coupled to the common base structure (114) and a holder portion (116A, 300A) holding the respective quadrupole magnet (112A-C), wherein the mount portion (116B, 300B) and the holder portion (116A, 300A) are kinematically and, optionally, removably coupled via a set of moveable kinematic mount points (118, 118A, 118B) that allow for adjusting the position and the orientation of the respective quadrupole magnet (112A-C) with respect to the mount portion (116B, 300B).

5. The transportable beamline (100, 200) of claim 4, wherein:
said mount portion (116B, 300B) comprises a set of height-adjustable pins (330), in particular height-adjustable threaded pins, forming said set of mount points (118, 118B) to allow for adjusting the position of the respective quadrupole magnet (112A-C) in a first direction (Y) and the orientation of the respective quadrupole magnet (112A-C) about a second direction (X) and a third direction (Z) perpendicular to the first direction (Y); and/or
said mount portion (116B, 300B) comprises a bottom part (328) coupled to the common base structure (114) and an upper part (326) that forms and/or supports said set of mount points (118, 118B), the upper part (326) being slidably arranged on the bottom part (328) to allow for adjusting the position of the respective quadrupole magnet (112A-C) in the second and third directions (X, Z) and the orientation of the respective quadrupole magnet (112A-C) about the first direction (Y), wherein the bottom and upper parts (326, 328) are configured to be locked to each other to prevent sliding of the upper part (326) relative to the bottom part (328), wherein optionally the transportable beamline (100, 200) further comprises a mechanical alignment template (900) having a plurality of precision holes (908A, 908B) and/or recesses arranged in a pattern corresponding to nominal positions of at least a subset of the set of mount points (118, 118B) for each of said some or all of said adjustable kinematic mounts (116, 300), each of said precision holes (908A, 908B) and/or recesses being configured to receive a respective one of the height-adjustable pins (330) for mechanical alignment of the set of mount points (118, 118B) of each of said some of all of said adjustable kinematic mounts (116, 300) with respect to each other; and/or
some or all of said adjustable kinematic mounts (116, 300) comprise an intermediate portion (116C, 300C) arranged between the mount and holder portions (116A/B, 300A/B), wherein said set of mount points (118, 118B) is a first set of moveable kinematic mount points (118B), the mount portion (116B, 300B) and the intermediate portion (116C, 300C) are kinematically and, optionally, removably coupled at said first set of moveable kinematic mount points (118B) and the intermediate portion (116C, 300C) and the holder portion (116A, 300A) are kinematically and, optionally, removably coupled at a second set of moveable kinematic mount points (118A) on the intermediate portion (116C, 300C) that allow for adjusting the position and the orientation of the respective quadrupole magnet (112A-C) with respect to the intermediate portion (116C, 300C), wherein optionally said intermediate portion (116C, 300C) comprises a base (310) arranged on the first set of mount points (118B) and a tip/tilt plate (308) that forms and/or supports said second set of mount points (118A), the tip/tilt plate (308) being moveably supported by the base (310) so as to be tiltable and translatable with respect to the base (310) in three directions.

6. The transportable beamline (100, 200) of any one of the preceding claims, wherein some or all of said two or more quadrupole magnets (112A-C) are mounted to a translation stage (214, 216), in particular an automated computer-controlled translation stage, on the common base structure (114) that is configured to move the respective magnet (112A-C) longitudinally along the beam path (104) relative to the common base structure (114), in particular wherein:
said two or more quadrupole magnets (112A-C) comprise an upstream quadrupole magnet (112A), a central quadrupole magnet (112C) and a downstream quadrupole magnet (112B) arranged along the beam path (104); and the focusing assembly (112) comprises a first translation stage (214) to which the upstream and central quadrupole magnets (112A, 112C) are mounted, the first translation stage (214) being configured to move the upstream and central quadrupole magnets (112A, 112C) longitudinally along the beam path (104) relative to the collimator (110, 400) and the downstream quadrupole magnet (112B) for adjusting an upstream drift length (Do) between the collimator (110, 400) and the upstream quadrupole magnet (112A) and a downstream drift length (D2) between the central quadruple magnet (112C) and the downstream quadrupole magnet (112B) while maintaining a central drift length (D1) between the upstream and central quadrupole magnets (112C, 112B), wherein optionally the transportable beamline further comprises a second translation stage (218, 220) to which the downstream quadrupole magnet (112B) is mounted, the second translation stage (218, 220) being configured to move the downstream quadrupole magnet (112B) laterally with respect to the beam path (104) for scanning the target point (106) perpendicular to the beam path (104); and/or
the transportable beamline (100, 200) further comprises a third translation stage (216) to which the central quadrupole magnet (112C) is mounted, the third translation stage (216) being mounted to the first translation stage (214) and configured to move the central quadrupole magnet (112C) longitudinally along the beam path (104) relative to the upstream quadrupole magnet (112A) for adjusting the central drift length (D1) and the downstream drift length (D2) simultaneously.

7. The transportable beamline (100, 200) of any one of the preceding claims, wherein:
said two or more quadrupole magnets (112A-C) are or comprise permanent magnets, in particular samarium-cobalt magnets; and/or
the transportable beamline (100, 200) further comprises one or more radiation shielding plates (222) arranged at an upstream entrance facet of a respective one of the two or more quadrupole magnets (112A-C); and/or
the energy degrader (108, 500) comprises one or more degrading members (108A/B, 502A/B) arranged or arrangeable in the beam path (104) for the ion beam (102) to pass therethrough, the one or more degrading members (108A/B, 502A/B) preferably comprising or consisting of boron carbide; and/or
the energy degrader (108, 500) is configured to decelerate ions in the ion beam (102) having an initial energy between 20 MeV/mᵤ and 100 MeV/mᵤ to a target energy between 10 MeV/mᵤ and 60 MeV/mᵤ with mᵤ denoting the mass of the ions in atomic mass units, in particular wherein the energy degrader (108, 500) is configured to decelerate ions in the ion beam (102) having an initial energy between 70 MeV/mᵤ and 100 MeV/mᵤ to a target energy between 20 MeV/mᵤ and 50 MeV/mᵤ and/or to decelerate ions in the ion beam (102) having an initial energy between 30 MeV/mᵤ and 50 MeV/mᵤ to a target energy between 10 MeV/mᵤ and 30 MeV/mᵤ; and/or
the energy degrader (108, 500) is an adjustable energy degrader configured to decelerate ions in the ion beam (102) from a fixed initial energy to an adjustable target energy.

8. The transportable beamline (100, 200) of any one of the preceding claims, wherein:
the collimator (110, 400) comprises a pair of apertures (110A, 110B, 400A/B), in particular adjustable apertures (400A, 400B) having an adjustable aperture size, wherein the apertures (110A, 110B, 400A, 400B) preferably comprise or consist of graphite; wherein optionally:
one or both of the apertures (110A, 110B, 400A, 400B) are each formed by one or more pairs of collimator plates (402) configured to move by a same distance in opposite directions for adjusting a size of the respective aperture (110A, 110B, 400A, 400B), in particular wherein for some or all of said pairs of collimator plates (402), the collimator plates (402) of the respective pair are mounted to translation stage slides (408) on an actuated double threaded spindle (410) for synchronously moving the collimator plates (402) in opposite directions; and/or
the collimator (110, 400) further comprises an elongated collimation tube (213) arranged between the pair of apertures (110A, 110B, 400A, 400B), the elongated collimation tube (213) preferably comprising or consisting of graphite; and/or
the transportable beamline (100, 200) further comprises a beam shaping aperture (224) arranged between the downstream-most quadrupole magnet (112B) along the beam path (104) and the closest quadrupole magnet (112C) upstream thereof along the beam path (104), wherein the beam shaping aperture (222) is configured to limit an extent of the ion beam (102) at least in a direction in which the downstream-most quadrupole magnet (112B) is defocusing.

9. The transportable beamline (100, 200) of any one of the preceding claims, further comprising a housing (202) in which the energy degrader (108, 500) and the common base structure (114) with the collimator (110, 400) and the focusing assembly (112) are arranged, wherein the housing (202) is configured to one or both of hold helium at atmospheric pressure and hold a vacuum of less than 0.5 mbar, preferably of less than 0.1 mbar, in particular wherein:
the housing (202) comprises an upstream entrance window (204) and a downstream exit window (208), in particular wherein the entrance and exit windows (204, 208) are formed of a polyimide and/or polyethylene terephthalate film, the polyimide and/or polyethylene terephthalate film preferably having a thickness of less than 200 µm, more preferably of less than 100 µm, most preferably of less than 20 µm, wherein optionally the transportable beamline (100, 200) further comprises an ionization-based monitoring chamber for monitoring the ion beam (102), said monitoring chamber sharing the entrance window (204) or the exit window (208) with the housing (202).

10. The transportable beamline (100, 200) of any one of the preceding claims, wherein:
the beamline (100, 200) is mounted or mountable to an adjustable supporting platform (600), in particular an adjustable optical table, configured to adjust a position and/or an orientation of the beamline (100, 200) for aligning the beam path (104) of the beamline (100, 200) to the ion beam (102) of the ion beam source; and/or
the common base structure (114) is mounted moveably in the beamline (100, 200) for moving the collimator (110, 400) and the focusing assembly (112) into and out of the beam path (104) while preserving relative alignment between the collimator (110, 400) and the two or more quadrupole magnets (112A-C).

11. The transportable beamline (100, 200) of any one of the preceding claims, wherein:
the beamline (100, 200) is configured to switch between a focused irradiation mode, in which the collimator (110, 400) and the focusing assembly (112) are arranged in the beam path (104), and a secondary irradiation mode, in which the focusing assembly (112) or one or more parts thereof and, optionally, the collimator (110, 440) are not arranged in the beam path (104), wherein preferably the beamline (100, 200) further comprises an additional collimator for imaging and/or passively collimated beam delivery, in particular a multi-slit collimator for minibeam irradiation and/or a compensator and/or 3D modulator for passively scattered beam delivery and/or FLASH beam delivery; and/or
the transportable beamline (100, 200) further comprises a scattering foil (516) arrangeable in the beam path (104) for the ion beam (102) to pass therethrough.

12. A method (800) of aligning a transportable beamline (100, 200) according to any one of the preceding claims, the method (800) comprising:
mounting the collimator (110, 400) and the focusing assembly (112) to the common base structure (114);
for each of the two or more quadrupole magnets (112A-C), aligning a magnetic axis of the quadrupole magnet (112A-C) to the beam path (104) for the ion beam (102) by adjusting a position and/or an orientation of the quadrupole magnet (112A-C) relative to the common base structure (114); and
fixing the position and the orientation of each of the two or more quadruple magnets (112A-C) relative to the common base structure (114).

13. The method (800) of claim 12, wherein some or all of said two or more quadrupole magnets (112A-C) are mounted to the common base structure (114) via a respective adjustable kinematic mount (116, 300) comprising a set of mount points (118, 118A, 118B) and aligning the magnetic axes of the respective quadrupole magnets (112A-C) to the beam path (104) for the ion beam (102) comprises adjusting at least a first mount point of the set of mount points (118, 118A, 118B) of the respective adjustable kinematic mount (116, 300), in particular wherein:
aligning the magnetic axes of said two or more quadrupole magnets (112A-C) comprises, for each of the two or more quadrupole magnets (112A-C), adjusting the first mount point of the respective adjustable kinematic mount (116, 300) to a nominal height above the common base structure (114) along a first lateral direction (Y) perpendicular to the beam path (104); and subsequently, for each of the two or more quadrupole magnets (112A-C), adjusting the remaining mount points of the set of mount points (118, 118A, 118B) of the respective adjustable kinematic mount (116, 300) to the nominal height above the common base structure (114); and/or
aligning the magnetic axes of said two or more quadrupole magnets (112A-C) comprises, for a first one and a second one of said two or more quadrupole magnets (112A-C), adjusting the first mount point of the respective adjustable kinematic mount (116, 300) to its nominal position along the beam path (104) and a second lateral direction (X), the second lateral direction (X) being perpendicular to the beam path (104) and to the first lateral direction (Y); and subsequently, for each of the two or more quadrupole magnets (112A-C), adjusting the remaining mount points (118, 118A, 118B) of the respective adjustable kinematic mount (116, 300) to their nominal positions along the beam path (104) and the second lateral direction (X), wherein optionally the first quadrupole magnet is the upstream-most quadrupole magnet (112A) along the beam path (104) of said two or more quadrupole magnets (112A-C) and/or the second quadrupole magnet is the downstream-most quadrupole magnet (112B) along the beam path (104) of said two or more quadrupole magnets (112A-C).

14. The method (800) of claim 13, wherein:
the mount points (118, 118A, 118B) of the adjustable kinematic mounts (116, 300) are adjusted using a mechanical alignment template (900) having a plurality of precision holes (908A, 908B) and/or recesses arranged in a pattern corresponding to the nominal positions of at least a subset of the mount points (118, 118A, 118B) for each of said adjustable kinematic mounts (116, 300), each of said precision holes (908A, 908B) and/or recesses being configured to receive a respective one of the mount points (118, 118A, 118B) of the adjustable kinematic mounts (116, 300) for mechanical alignment of the mount points (118, 118A, 118B) of the adjustable kinematic mounts (116, 300) with respect to each other; and/or
some or all of said adjustable kinematic mounts (116, 300) each comprise a mount portion (116B, 300B) for coupling to the common base structure (114), a holder portion (116A, 300A) holding the respective quadrupole magnet (112A-C) and an intermediate portion (116C, 300C) arranged between the mount and holder portions (116A/B, 300A/B), wherein for each of said some or all of said adjustable kinematic mounts (116, 300), said set of mount points (118, 118A, 118B) is a first set of mount points (118B) coupling the mount portion (116B, 300B) and the intermediate portion (116C, 300C) of the respective adjustable kinematic mount (116, 300) and the respective adjustable kinematic mount (116, 300) further comprises a second set of mount points (118A) coupling the intermediate portion (116C, 300C) and the holder portion (116A, 300A), and the method (800) further comprises, for at least one of said some or all of said adjustable kinematic mounts (116, 300), adjusting one or more mount points of the second set of mount points (118A) of the respective adjustable kinematic mount (116, 300) to align the magnetic axis of the respective quadrupole magnet (112A-C) relative to the intermediate portion (116C, 300C) of the adjustable kinematic mount (116, 300) and subsequently fixing the position and the orientation of the quadruple magnet (112A-C) relative to the intermediate portion (116C, 300C).

15. The method (800) of any one of claims 12 to 14, further comprising:
aligning the collimator (110, 400) relative to the magnetic axes of said two or more quadrupole magnets (112A-C), in particular wherein aligning the collimator (110, 400) relative to the magnetic axes of said two or more quadrupole magnets (112A-C) comprises aligning an optical axis of an optical telescope to the magnetic axes of the two or more quadrupole magnets (112A-C) and aligning the collimator (110, 400) to the optical axis of the telescope; and/or
mounting the beamline (100, 200) to a supporting platform (600), in particular an optical table, and adjusting a position and/or an orientation of the supporting platform (600) to align the beam path (104) of the beamline (100, 200) to an ion beam (102) of an ion beam source.
